# EUROPEAN PATENT APPLICATION

(11) **EP 4 650 438 A1**
(43) Date of publication of application: **19.11.2025**
(21) Application number: 24175778.0
(22) Date of filing: 14.05.2024
(51) Int. Cl.: C12N 5/077

(54) **CELLS HAVING THE PHENOTYPE OF CARDIAC VALVE CELLS AND THE TREATMENT OF A VALVE DEFECT**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner Patentanwälte Rechtsanwälte mbB

(57) **Abstract**

The present invention relates to a method of producing cells having the phenotype of cardiac valve cells from precursor cells of valve cells comprising (a) introducing into said precursor cells early growth response 3 (EGR3) and/or a nucleic acid sequence encoding *EGR3.* The present invention also relates to a valve transplant comprising cells having the phenotype of valve cells obtained or obtainable by the method of the invention. The present invention furthermore relates to cells having the phenotype of valve cells obtained or obtainable by the method of the invention, the valve transplant of the invention or a compound promoting the expression and/or the activity of early growth response 3 (EGR3) for use in treating a subject having a valve defect.

## Description

The present invention relates to a method of producing cells having the phenotype of cardiac valve cells from precursor cells of valve cells comprising (a) introducing into said precursor cells early growth response 3 (*EGR3*) and/or a nucleic acid sequence encoding *EGR3.* The present invention also relates to a valve transplant comprising cells having the phenotype of valve cells obtained or obtainable by the method of the invention. The present invention furthermore relates to cells having the phenotype of valve cells obtained or obtainable by the method of the invention, the valve transplant of the invention or a compound promoting the expression and/or the activity of early growth response 3 (EGR3) for use in treating a subject having a valve defect.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The heart consists of four chambers, two atria (upper chambers) and two ventricles (lower chambers). There is a cardiac valve (or heart valve) through which blood passes before leaving each chamber of the heart. The cardiac valves prevent the backward flow of blood. These valves are actual flaps that are located on each end of the two ventricles (lower chambers of the heart). They act as one-way inlets of blood on one side of a ventricle and one-way outlets of blood on the other side of a ventricle. Normal valves have three flaps, except the mitral valve, which has two flaps. The four heart valves include the following: tricuspid valve: located between the right atrium and the right ventricle; pulmonary valve: located between the right ventricle and the pulmonary artery; mitral valve: located between the left atrium and the left ventricle and aortic valve: located between the left ventricle and the aorta.

Cardiac valves can have malfunctions. Regurgitation (or leakage of the valve): The valve(s) does not close completely, causing the blood to flow backward through the valve. This results in leakage of blood back into the atria from the ventricles (in the case of the mitral and tricuspid valves) or leakage of blood back into the ventricles (in the case of the aortic and pulmonary valves). Stenosis (or narrowing of the valve): The valve(s) opening becomes narrowed or valves become damaged or scarred (stiff), inhibiting the flow of blood out of the ventricles or atria. The heart is forced to pump blood with increased force in order to move blood through the narrowed or stiff (stenotic) valve(s). Prolapse: The valve flaps become stretched out and floppy. They bulge backward like a parachute. This condition can lead to regurgitation. Atresia: The valve isn't formed. A solid sheet of tissue blocks the blood flow between the heart chambers. This type usually affects the pulmonary valve.

Congenital valve diseases, such as bicuspid aortic valve and pulmonary atresia, appear during early development and affect the structural integrity and functionality of heart valves (Hinton et al. (2011). Annu Rev Physiol.; 73:29-46). In contrast, adult populations are more commonly affected by conditions such as valve calcification, stenosis, and regurgitation, which contribute to compromised valve performance and are often the result of degenerative processes.

Cardiac valves can have more than one malfunction at the same time (e.g., regurgitation and stenosis). Also, more than one heart valve can be affected at the same time. When cardiac valves fail to open and close properly, the implications for the heart can be serious, possibly hampering the heart's ability to pump blood adequately through the body. Cardiac valve problems are one cause of heart failure, stroke, blood clots, heart rhythm abnormalities and even death.

Heart valve disease can be treated by medicaments or surgery. Medicaments are not a cure for heart valve disease, but treatment can often relieve symptoms. Surgery can be used to repair or replace the malfunctioning valve(s). Surgery may include heart valve repair or heart valve replacement.

The current therapeutic landscape includes valve transplants from donors, animal-derived valves, and prosthetic valves, as well as less invasive transcatheter approaches (Goel et al. (2020)., Current opinion in cardiology; 35(4):313-8). Despite their utility, issues such as the potential for immune rejection and the finite lifespan of prosthetic materials limit these strategies. Alternative technologies, such as biopolymer casts and Ventricular Assist Devices (VADs), alongside advances in stem cell biology, such as the development of feeder-free induced pluripotent stem cell (iPSC) differentiation techniques into valve cells and human organoids, represent significant strides toward addressing these limitations (Neri et al. (2019), Nat Commun; 10(1):1929; Cheng et al. (2021), Commun Biol; 4(1):1039; Liu et al. (2024), iScience; 27(1):108599; Ming et al. (2022), Biosens Bioelectron; 207:114136; and Volmert et al. (2023), Nat Commun; 14(1):8245).

However, current *in vitro* valve differentiation protocols require complex combinations of factors to form valve tissues, and no reagent exists so far to differentiate valve cells *in vivo* (Thomas et al. (2022). BMC Cardiovasc Disord; 22(1):122).

Congenital heart disease is the most common congenital disorder in newborns, with an incidence of approximately 1% of live births (van der Bom et al. (2011), Nat Rev Cardiol; 8(1):50-60). 2.5% of Americans live with moderate to severe valvular heart disease and 180,000 of them undergo heart valve replacement surgeries each year (Coffey et al. (2021), Nat Rev Cardiol; 18(12):853-64). Consequently, valve defects resulting from congenital or acquired diseases pose significant challenges to cardiovascular health and require improvements in therapeutic strategies.

Hence, while already some treatment options for heart valve diseases are available there is an ongoing need to improve the treatment options and/or to provide further treatment options. This need is addressed by the present invention.

Accordingly, the present invention relates in a first aspect a method of producing cells having the phenotype of cardiac valve cells from precursor cells of valve cells comprising (a) introducing into said precursor cells early growth response 3 (EGR3) and/or a nucleic acid sequence encoding *EGR3* (thereby differentiating said precursor cells into cells having a phenotype of cardiac valve cells).

The method is preferably an *in vitro* or *ex vivo* method.

Cardiac valves are composed of valvular endothelial cells (VECs; an endothelial cell type) that line the surfaces of the leaflets, and valvular interstitial cells (VICs; a mesenchymal cell type) distributed throughout the leaflets. Both VECs and VICs maintain tissue homeostasis for the day-today function of cardiac valves, as they secrete biochemical signals, matrix proteins, and matrix remodeling enzymes (Wang et al. (2014), Nat Rev Cardiol. 2014 Dec; 11(12): 715-727). The examples show that Egr3 is a differentiation factor for both, VICs and VECs. *egr3* mutant zebrafish fail to form valve leaflets as their endocardial cells remain as a monolayer in a fully penetrant fashion.

Both, VICs and VECs express the marker genes SOX9, POSTN (Periostin), HEY2, TGFB2 and HAND2. Accordingly, cells having the phenotype of cardiac valve cells express with increasing preference one or more, two or more, three or more, four or more or all five of SOX9, POSTN, HEY2, TGFB2, and HAND2.

VICs express the marker genes VIM (Vimentin), CD44, CD90, CD105, SPP1, α-SMA, SOX9, N-CAD, CDH11, VIM, COL1A1, COL3A1, and POSTN. Accordingly in a further embodiment, cells having the phenotype of cardiac valve cells express with increasing preference one or more, two or more, three or more, four or more, five or more, six or more, seven or more, eight or more, nine or more, ten or more, eleven or more, twelve or more and all thirteen of VIM, CD44, CD90, CD105, SPP1, α-SMA, SOX9, N-CAD, CDH11, VIM, COL1A1, COL3A1, and POSTN.

VECs express the marker genes NAFTc1, WBT2, BMP4, NFκB1, CCND1, SMAD1, NPR3, CDH11, EDN1, GATA5, MEF2C, SOX17, THSD1, EMCN, NOTCH4, DLL3/4 and JAG1 (Cheng et a. (2021), Communications Biology, 4:Art.No. 1039). Accordingly in an alternative embodiment, cells having the phenotype of cardiac valve cells express with increasing preference one or more, two or more, three or more, four or more, five or more, six or more, seven or more, eight or more, nine or more, ten or more, eleven or more, twelve or more, thirteen or more, fourteen or more, fifteen or more, sixteen or more and all seventeen of NAFTc1, WBT2, BMP4, NFκB1, CCND1, SMAD1, NPR3, CDH11, EDN1, GATA5, MEF2C, SOX17, THSD1, EMCN, NOTCH4, DLL3/4 and JAG1.

The cells having the phenotype of cardiac valve cells may also be designated cardiac valve-like cells. Cells having the phenotype of cardiac valve cells are not identical to naturally occurring cardiac valve cells since they are generated *in vitro* / *ex vivo*, but shall as closely as possible resemble naturally occurring cardiac valve cells.

The precursor cells (or progenitor cells) of valve cells can be any cells that can be further differentiated into cells having the phenotype of cardiac valve cells by introducing into said precursor cells early growth response 3 (EGR3) and/or a nucleic acid sequence encoding *EGR3.* Thus, a precursor cell specifies a cell which is partially or fully undifferentiated. With regard to the present invention, the precursor cell is a partially differentiated cell or a fully undifferentiated cell and has the capability to differentiate into a cell having the phenotype of cardiac valve cells. Preferred examples will be described herein below.

Early growth response 3 (EGR3) is a transcriptional regulator that belongs to the EGR family of C2H2-type zinc-finger proteins. It is an immediate-early growth response gene which is induced by mitogenic stimulation.

Early growth response 3 (EGR3) preferably comprises or consists of an amino acid sequence which comprises or consists of the nucleic acid sequence of SEQ ID NO: 1 or 3, or an amino acid sequence which is at least 80%, preferably at least 90% and most preferably at least 95% identical thereto.

The nucleic acid sequence encoding *EGR3* preferably comprises or consists of a nucleic acid sequence which comprises or consists of the nucleic acid sequence of SEQ ID NO: 2 or 4, or a nucleic acid sequence which is at least 80%, preferably at least 90% and most preferably at least 95% identical thereto.

Also envisioned herein are with more increasing preference sequence identities of least 96%, at least 97% and at least 98%, at least 99% and 100%.

Further details on SEQ ID NOs 1 to 4 will be provided herein below in connection with the second aspect of the invention. These details apply *mutatis mutandis* to the first aspect of the invention.

The term "nucleic acid molecule", in accordance with the present invention, includes DNA, such as cDNA or genomic DNA, and RNA. It is understood that the term "RNA" as used herein comprises all forms of RNA including mRNA. The term "nucleic acid molecule" is interchangeably used in accordance with the invention with the term "polynucleotide".

The nucleic acid molecule(s) may also comprise regulatory regions or other untranslated regions. In a further embodiment, said nucleic acid molecule may comprise heterologous nucleic acid which may encode heterologous proteinaceous material thus giving rise, e.g., to fusion proteins.

The present invention, thus, relates to a nucleic acid molecule(s) of the invention encoding a fusion protein. For example, the nucleic acid molecule of the invention can be fused in frame to a detectable marker such as purification marker or a direct or indirect fluorescence marker. Purification marker comprise one or more of the following tags: His, lacZ, GST, maltose-binding protein, NusA, BCCP, c-myc, CaM, FLAG, GFP, YFP, cherry, thioredoxin, poly(NANP), V5, Snap, HA, chitin-binding protein, Softag 1, Softag 3, Strep, or S-protein. Suitable direct or indirect fluorescence marker comprise FLAG, GFP, YFP, RFP, dTomato, cherry, Cy3, Cy 5, Cy 5.5., Cy 7, DNP, AMCA, Biotin, Digoxigenin, Tamra, Texas Red, rhodamine, Alexa fluors, FITC, TRITC or any other fluorescent dye or hapten.

The term "introducing" as used herein specifies the process of bringing a protein and/or nucleic acids sequence into a living cell, an in particular also into the cell nucleus, preferably by introducing means as defined herein below. In accordance with the invention, the protein to be introduced is EGR3 and the nucleic acid sequences is the nucleic acid sequence encoding *EGR3.*

The method of the invention is carried out under suitable cell culture conditions. It is of note that cell culture conditions for a cell population comprising precursor cells of valve cells are known from the prior art (e.g. Neri et al. (2019), Nat Commun; 10(1):1929; Cheng et al. (2021), Commun Biol; 4(1):1039; Liu et al. (2024), iScience; 27(1):108599; Ming et al. (2022), Biosens Bioelectron; 207:114136; and Volmert et al. (2023), Nat Commun; 14(1):8245).

In the appended examples zebrafish was a model system for human heart valves. Zebrafish heart valves share high physiological and signaling similarities with human valves, making this model highly relevant for studying valve disease mechanisms (Paolini and Abdelilah-Seyfried (2018), Curr Opin Cell Biol; 55:52-8). In the zebrafish heart valves it was surprisingly found that the expression of Egr3induced cardiac valve cell differentiation in a whole-organism context. *egr3* is only expressed in valve cells in endothelial tissues and *egr3* loss-of-function suppresses valve formation. Egr3 overexpression alone in the endocardium is sufficient to promote *de novo* valve identity in the endocardium. The acquisition of valve identity following *egr3* overexpression also occurs in flow defective conditions, bypassing its requirement for valve differentiation. The surprising finding that Egr3 as a single differentiation factor mediates cardiac valve cell differentiation was confirmed in mammalian hearts based on the pathological condition of Left Ventricular Assist Device (LVAD) in human. It is shown in the appended examples that egr3 is also expressed in the valve region of mammalian hearts and that *Egr3* is upregulated in porcine valve cells subject to physiological patterns of flow stimulation. Moreover, it is shown that the aortic valves of VAD patients, present an upregulation of *EGR3,* following a biomechanical overload. It is furthermore shown in the examples that EGR3 overexpression is sufficient to induce valve differentiation in human umbilical vein endothelial cells (HUVEC). These data show that *egr3* is a suitable means for producing cells having the phenotype of cardiac valve cells from precursor cells of valve cells, which *inter alia* allows for the production of valve transplants. In addition, the data show that the cells having the phenotype of cardiac valve cells, the valve transplants and Egr3/ EGR3 can be used to treat valve defects. Thus, data in appended examples provides a novel pathway to overcome the limitations of existing valve defect treatment, in particular valve replacement therapies.

In accordance with a preferred embodiment of the first aspect the method comprises prior to step (a), (a') providing a cell population comprising precursor cells of valves cells, wherein said cell population has been obtained from a subject or has been derived from a cell line.

The cell population might have been obtained from a subject or might have been derived from a cell line.

In accordance with a further preferred embodiment the method comprises after step (a), (b) isolating cells having the phenotype of valve cells from the cell population obtained in step (a).

Methods for isolating cells are well known in the state of the art and comprise FACS (fluorescence activated cell sorting), sucrose gradient centrifugation, (laser) microdissection, single cell dilution, and Magnetic Labeled Bead Cell Separation. FACS is a method for sorting a heterogeneous mixture of biological cells into two or more containers, one cell at a time, based upon the specific light scattering and fluorescent characteristics of each cell. For this purpose a cell specific marker, e.g. a valve-specific cell-surface marker, such as PECAM-1 and VCAM-1 may be labeled with a fluorescent dye like FLAG, GFP, YFP, RFP, dTomato, cherry, Cy3, Cy 5, Cy 5.5., Cy 7, AMCA, Tamra, Texas Red, rhodamine, Alexa fluors, FITC or TRITC. Sucrose gradient centrifugation is a centrifugation technique which separates compounds according to their density along a sucrose density gradient which is typically created overlaying lower concentrations of sucrose on higher concentrations in a centrifuge tube. Single cell dilution is a technique of diluting cells, preferably in culture medium until a concentration is reached that allows to separate single cell in a separated vial. Magnetic Labeled Bead Cell Separation is a commercial system available from Miltenyi Biotec (MACS), Bergisch Gladbach, Germany.

In accordance with another preferred embodiment introducing into said precursor cells is effected by (i) transforming said precursor cells with a nucleic acids molecule comprising the nucleic acid sequence encoding *EGR3*; and/or (ii) microinjection, electroporation, lipofection and/or protein transduction of *EGR3* into said precursor cells.

Methods for transformation of cells with a nucleic acids molecule are well established in the state of the art and include but are not limited to viral transformation (e.g. adenoviral, adeno-associated, retroviral, lentiviral transfection), transposon transformation (e.g. retrotransposons, DNA-transposons, retroviruses as transposable elements, and in particular Tc1/mariner-class transposons like piggyBac and Sleeping Beauty), lipofection, microinjection, electroporation, impalefection, gene gun, magnetofectin, sono-poration, optical transfection (e.g. by a laser), and chemical-based transfection.

These methods for introducing a protein (or peptide) directly into a cell are well known in the state of the art and comprise but are not limited to microinjection, electroporation, lipofection (using liposomes) and protein transduction. In this regard, the proteins to be introduced may either be isolated form their natural environment or recombinantly produced. These methods are capable to bypass or further support the step of transforming a cell as described herein above.

A liposome used for lipofection is a small vesicle, composed of the same material as a cell membrane (i.e., normally a lipid bilayer e.g., out of phospholipids), which can be filled with one more protein(s) (e.g., Torchilin VP. (2006), Adv Drug Deliv Rev., 58(14):1532-55). To deliver a protein into a cell, the lipid bilayer of the liposome can fuse with the lipid bilayer of the cell membrane, thereby delivering the contained protein into the cell. It is preferred that the liposomes used in accordance with invention are composed of cationic lipids. The cationic liposome strategy has been applied successfully to protein delivery (Zelphati et al. (2001). J. Biol. Chem. 276, 35103-35110). As known in the art, the exact composition and/or mixture of cationic lipids used can be altered, depending upon the protein(s) of interest and the cell type used (Felgner et al. (1994). J. Biol. Chem. 269, 2550-2561).

Protein transduction specifies the internalisation of proteins into the cell, from the external environment (Ford et al (2001), Gene Therapy, 8:1-4). This method relies on the inherent property of a small number of proteins and peptides (preferably 10 to 16 amino acids long) being able to penetrate the cell membrane. The transducing property of these molecules can be conferred upon proteins which are expressed as fusions with them and thus offers, for example, an alternative to gene therapy for the delivery of therapeutic proteins into target cells. Commonly used proteins or peptides being able to penetrate the cell membrane are, for example; the antennapedia peptide, the herpes simplex virus VP22 protein, HIV TAT protein transduction domain, peptides derived from neurotransmitters or hormones, or a 9xArg-tag.

Microinjection and electroporation are well known in the art and the skilled person knows how to perform these methods. Microinjection refers to the process of using a glass micropipette to introduce substances at a microscopic or borderline macroscopic level into a single living cell. Electroporation is a significant increase in the electrical conductivity and permeability of the cell plasma membrane caused by an externally applied electrical field. By increasing permeability protein (or peptides or nucleic acid).

In accordance with a more preferred embodiment the nucleic acids molecule is a vector.

Accordingly, the present invention also relates to a vector containing the nucleic acid molecule(s) of the present invention. Preferably, the vector is a plasmid, cosmid, virus, bacteriophage or another vector used e.g. conventionally in genetic engineering.

The nucleic acid molecule may be inserted into several commercially available vectors. The nucleic acid molecule(s) referred to above may also be inserted into vectors such that a translational fusion with another polynucleotide is generated. The other polynucleotide may encode a protein which may e.g. increase the solubility, correct protein folding, facilitate the purification of the cells of the invention and/or further differentiation factors enhancing differentiation and/or maintenance of the valve cell phenotype. For vector modification techniques, see Sambrook and Russel (2001), loc. Cit. Generally, vectors can contain one or more origin of replication (ori) and inheritance systems for cloning or expression, one or more markers for selection in the host, e. g. dihydrofolate reductase, G418, neomycin, ampicillin, hygromycin, or kanamycin, and one or more expression cassettes. Suitable origins of replication (ori) include, for example, the Col E1, the SV40 viral and the M 13 origins of replication.

The coding sequences inserted in the vector can e.g. be synthesized by standard methods, or isolated from natural sources, such as pools of cDNA. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of transcription (e. g., translation initiation codon, promoters, such as naturally-associated or heterologous promoters and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers.

An expression vector according to this invention is capable of directing the replication, and the expression, of the polynucleotide and encoded differentiation factor(s) of this invention. Suitable expression vectors which comprise the described regulatory elements are known in the art. The nucleic acid molecules as described herein above may be designed for direct introduction, phage vectors or viral vectors (e.g., adenoviral, retroviral) into a cell.

A typical mammalian expression vector contains the promoter element, which mediates the initiation of transcription of mRNA, the protein coding sequence, and signals required for the termination of transcription and polyadenylation of the transcript and moreover, elements such as origin of replication, drug resistance gene, regulators (as part of an inducible promoter) may also be included. Highly efficient transcription can be achieved with the early and late promoters from SV40, the long terminal repeats (LTRs) from retroviruses, e.g., RSV, HTLVI, HIVI, and the early promoter of the cytomegalovirus (CMV). Alternatively, the recombinant polypeptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded polypeptide.

In accordance with an even more preferred embodiment the vector is a lentiviral, adenoviral or adeno-associated vector.

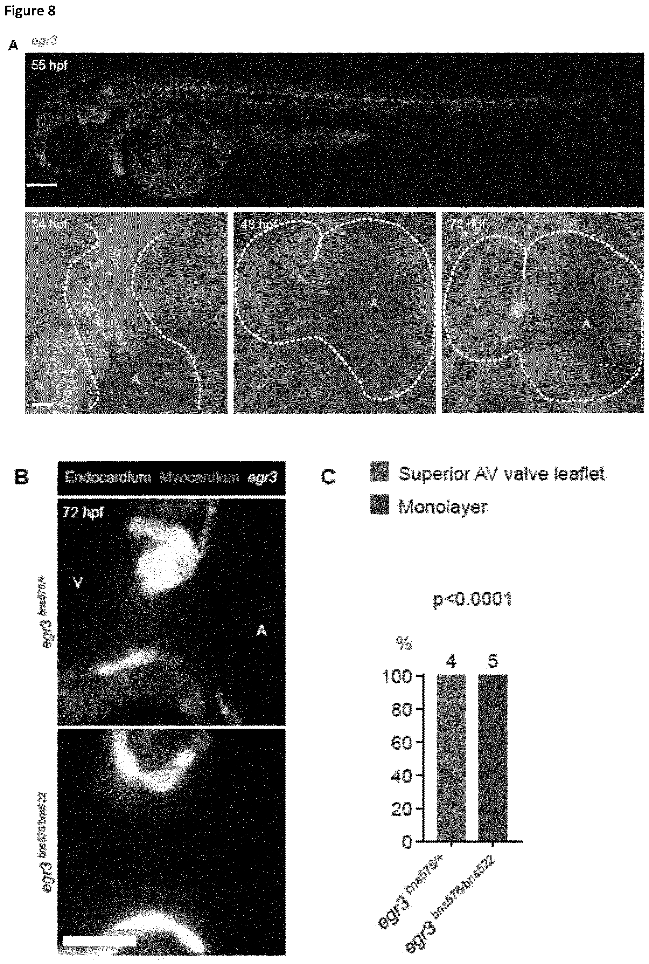

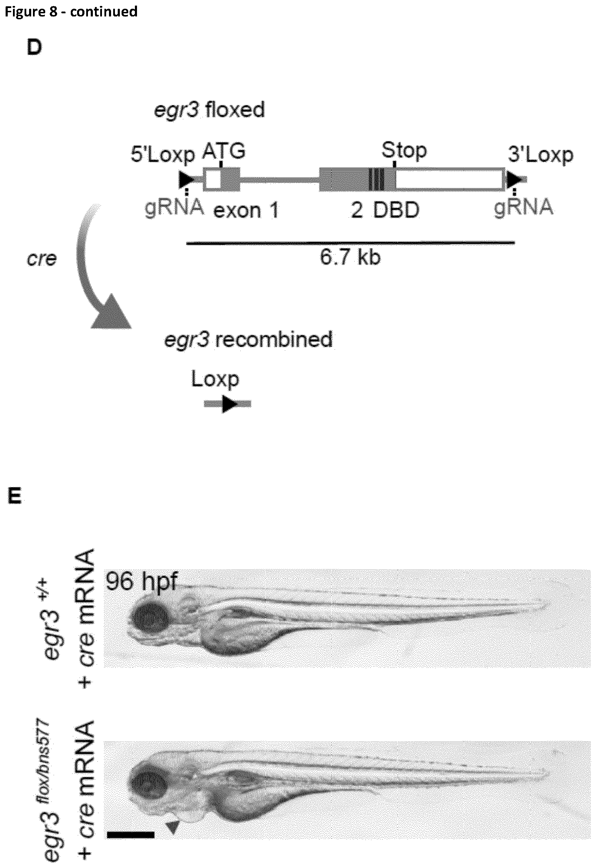

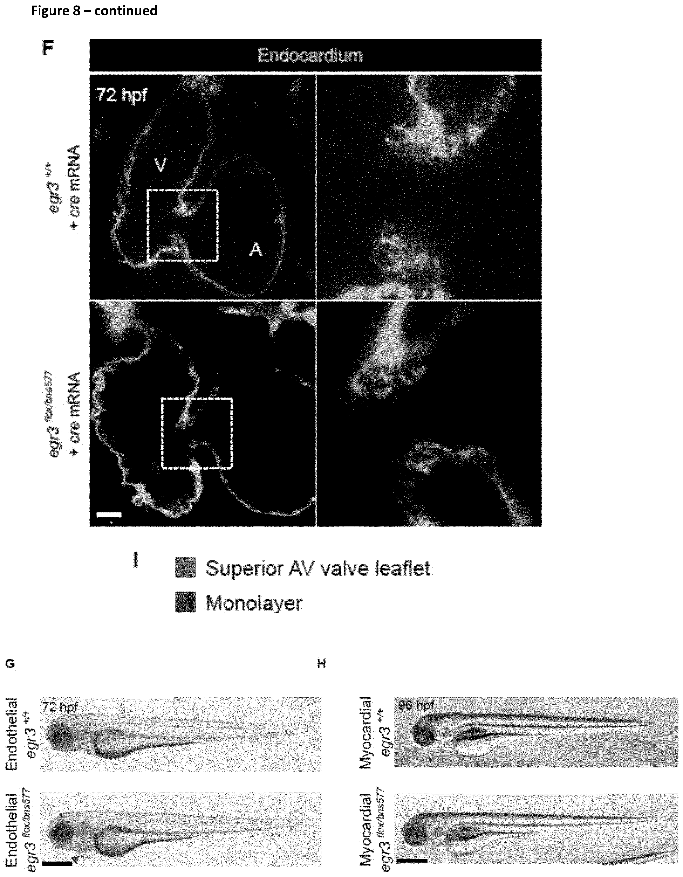

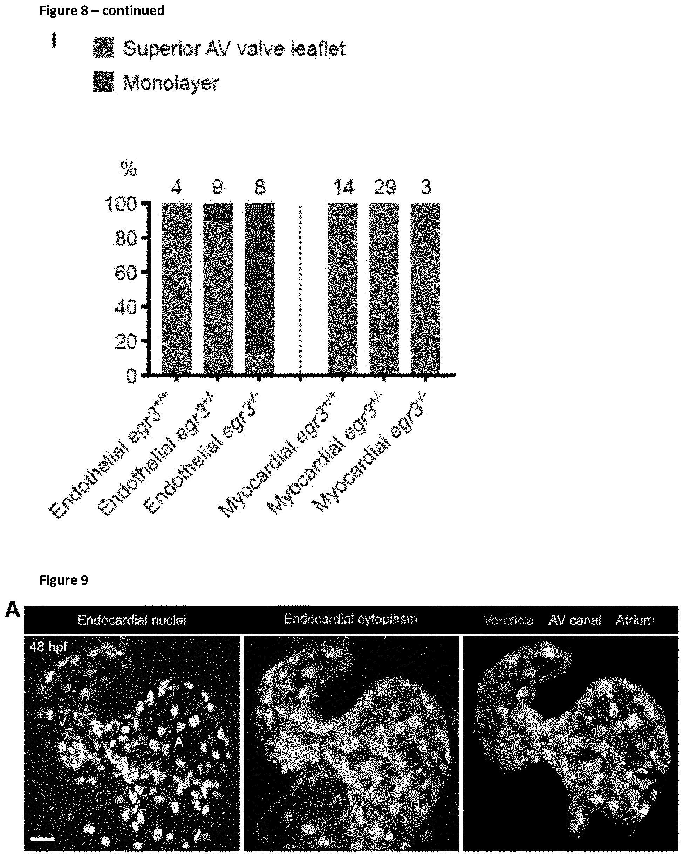

Examples of lentiviruses providing elements of a lentiviral vector are HIV, SIV, FIV, Puma lentivirus, EIA,·Bovine immunodeficiency virus, Caprine arthritis encephalitis virus, or Visna/maedi virus. Preferably used in accordance with the invention is a 3^{rd} generation lentiviral expression plasmid containing a SFFV promoter driven IRES-Puro (Foxa2), IRES-dTomato (C/EBPα) or IRES-eGFP (HNF4α) expression cassette. More preferably, one or more vectors as shown in Figures 8 to 10 are used. Lentiviruses are particularly useful in the method for the invention because they integrate into the genome and thus allow for non-transient gene expression. Moreover, studies have shown that lentivirus vectors have a lower tendency to integrate in places that potentially cause cancer than gamma-retroviral vectors which also integrate into the genome (Cattoglio et al. (2007), Blood, 110(6):1770-1778). Thus, they have a lower tendency of abnormal cell growth upon integration.

In accordance with a preferred embodiment the precursor cells are embryonic stem cells, adult stem cells, iPS cells (induced pluripotent stem cells) or endothelial cells (such as HUVEC cells).

Stem cells like embryonic stem cells, adult stem cells, or iPS cells are characterized by their ability to renew themselves through mitotic cell division and to differentiate into a diverse range of specialized cell types, including valve cells or valve-like cells. Accordingly, embryonic stem cells, adult stem cells, or iPS cells are particularly suitable precursor cells in accordance with the invention.

The embryonic stem cells as far as being human embryonic stem cells were not established by a process involving the step of disrupting a human embryo. In other words, the method of the invention is practiced without the need of disrupting a human embryo, by for example using human embryonic stem cell lines established and available at the time the invention was made. Such cells are inter alia described in Thomson et. al (1998), "Blastocysts Embryonic Stem Cell Lines Derived from Human", Science 282 (1145): 1145-1147, or Thomson et al. (1998), "Embryonic stem cell lines derived from human blastocysts", Science 282 (5391): 1145-7. Specific examples are the cell lines CHB-1, CHB-2, or CHB-3.

Also endothelial cells, such as and preferably HUVEC (Human Umbilical Vein Endothelial) cells can be precursor cells in accordance with the invention. HUVEC cells can be isolated from the vein of the umbilical cord of single, pooled or pre-screened donors. HUVECs can be easily made to proliferate in a laboratory setting and are therefore particularly preferred.

In a preferred embodiment of the method of the invention the obtained cells are free of pathogens.

The term "pathogen" in accordance with the invention refers to a biological agent that causes or contributes to a disease in the host. Examples of pathogens comprise viral pathogens, fungal pathogens, helminthic pathogens, prionic pathogens, protistic pathogens and bacterial pathogens.

In accordance with another preferred embodiment the cells having the phenotype of valve cells are (i) with increasing preference characterized by the expression of one or more, two or more, three or more, four or more, and all five of SOX9, POSTN, HEY2, TGFB2 and HAND2; and/or (ii) with increasing preference characterized by the expression of one or more, two or more, three or more, four or more, five or more, six or more, seven or more, eight or more, nine or more, ten or more, eleven or more, twelve or more and all thirteen of VIM, CD44, CD90, CD105, SPP1, α-SMA, SOX9, N-CAD, CDH11, VIM, COL1A1, COL3A1, and POSTN; and/or (iii) with increasing preference characterized by the expression of one or more, two or more, three or more, four or more, five or more, six or more, seven or more, eight or more, nine or more, ten or more, eleven or more, twelve or more, thirteen or more, fourteen or more, fifteen or more, sixteen or more and all seventeen of NFATc1, WBT2, BMP4, NFκB1, CCND1, SMAD1, NPR3, CDH11, EDN1, GATA5, MEF2C, SOX17, THSD1, EMCN, NOTCH4, DLL3/4 and JAG1.

As discussed herein above, SOX9, POSTN, HEY2, TGFB2 and HAND2 are expressed by VICS and VECs, VIM, CD44, CD90, CD105, SPP1, α-SMA, SOX9, N-CAD, CDH11, VIM, COL1A1, COL3A1, and POSTN by VICS, and NFATc1, WBT2, BMP4, NFκB1, CCND1, SMAD1, NPR3, CDH11, EDN1, GATA5, MEF2C, SOX17, THSD1, EMCN, NOTCH4, DLL3/4 and JAG1 by VECs. Accordingly, cells expressing these gene markers can be classified cells having the phenotype of valve cells.

In accordance with another preferred embodiment step (a) is carried out in the presence of one or more additional differentiation factors selected from the group consisting of BMP2, BMP10, bFGF, FGF2, FGF8, Wnt3a and VEGF.

BMP2 (bone morphogenetic protein 2), BMP10 (bone morphogenetic protein 10), bFGF (basic fibroblast growth factor), FGF2 (fibroblast growth factor 2), FGF8 (fibroblast growth factor 8), Wnt3a (Wnt gene family member 3a) and VEGF (vascular endothelial growth factor) are known differentiation factors that may aid or render EGR3 more efficient in differentiating said precursor cells into cells having a phenotype of cardiac valve cells.

Also, these additional differentiation factors may be used as proteins and/or as nucleic acid sequence encoding the proteins, just a described herein above for EGR3.

The present invention also relates to cells having the phenotype of valve cells obtained or obtainable by the method of the first aspect.

As discussed herein, the cells having the phenotype of valve cells obtained or obtainable by the method of the first aspect closely resemble but are not identical to naturally occurring valve cells, at least because they are generally produced *ex vivo* or *in vitro.*

The present invention relates in a second aspect to a valve transplant comprising cells having the phenotype of valve cells obtained or obtainable by the method of the first aspect.

The cells having the phenotype of valve cells obtained or obtainable by the method of the first aspect are particularly well suitable for the generation of a valve transplant.

The valve transplant is preferably an allograft transplant and most preferably an autologous transplant. An allograft valve transplant is a valve transplant, wherein the valve-like cells to be transplanted are from the same species as the recipient of the valve-like cells. An autologous valve transplant is a valve transplant, wherein the valve-like cells to be transplanted are derived from the recipient of the valve-like cells; i.e. from the same subject.

The species or subject herein is preferably a mammal. The mammal is preferably any one of cat, dog, horse, cattle, swine, goat, sheep, mouse, rat, monkey, ape and human, and most preferably a human.

The present invention relates in a second aspect to cells having the phenotype of valve cells obtained or obtainable by the method of the first aspect, the valve transplant of the second aspect or a compound promoting the expression and/or the activity of early growth response 3 (EGR3) for use in treating a subject having a valve defect.

Accordingly, also described herein is a method of treating or preventing a valve defect comprising administering a pharmaceutically effective amount of cells having the phenotype of valve cells obtained or obtainable by the method of the first aspect, the valve transplant of the second aspect or a compound promoting the expression and/or the activity of early growth response 3 (EGR3) to a subject having a valve defect.

Cells having the phenotype of valve cells, valve transplants comprising such cells and a compound promoting the expression and/or the activity of early growth response 3 (EGR3) are all suitable means for treating a subject having a valve defect. Depending on the severity of the valve defect, it might be necessary to replace the valves by valve transplants or it may be sufficient to treat the valve defect by valve cells obtained or obtainable by the method of the first aspect that substitute defective valve cells.

The treatment of a valve defect comprises the prophylactic and curative treatment and in preferably a curative treatment.

Moreover, also described herein is a method of treating or preventing a valve disease in a subject in need thereof comprising (a) isolating precursor cells of valve cells or a cell population comprising precursor cells of valve cells from the subject; (b) introducing into said precursor cells early growth response 3 (EGR3) and/or a nucleic acid sequence encoding *EGR3* thereby obtaining cells having the phenotype of valve cells; and (c) administering an pharmaceutically effective amount of the cells having the phenotype of valve cells obtained in (b) to said subject.

In accordance with a preferred embodiment of the second aspect the compound is early growth response 3 (EGR3) and/or a nucleic acid sequence encoding *EGR3.*

In accordance with this preferred embodiment the compound promoting the expression and/or the activity of early growth response 3 (EGR3) is the early growth response 3 (EGR3) protein and/or a nucleic acid sequence encoding *EGR3.* The nucleic acid sequence encoding *EGR3* preferably encodes EGR3 in an expressible form.

The term "in an expressible form" means that once the nucleic acid sequence encoding *EGR3* has been introduced into a cell, in particular a progenitor cell or a valve cell, *EGR3* mRNA is expressed from the nucleic acid sequence encoding *EGR3* and translated into EGR3.

In accordance with a preferred embodiment of the second aspect the compound is
(a) an amino acid sequence which comprises or consists of the nucleic acid sequence of SEQ ID NO: 1 or 3, or an amino acid sequence which is at least 80%, preferably at least 90% and most preferably at least 95% identical thereto,
(b) a nucleic acid sequence which comprises or consists of the nucleic acid sequence of SEQ ID NO: 2 or 4, or a nucleic acid sequence which is at least 80%, preferably at least 90% and most preferably at least 95% identical thereto,
(c) an expression vector expressing the nucleic acid sequence as defined in (b), preferably under the control of an endocardial -specific and/or valve-cell promoter, and
(d) a host cell comprising the expression vector of (c).

SEQ ID NO: 1 is the amnio acid sequence of the human EGR3 protein: 
MTGKLAEKLPVTMSSLLNQLPDNLYPEEIPSALNLFSGSSDSVVHYNQMATENVMDIGLTNEKPNPELSYSGSFQP
APGNKTVTYLGKFAFDSPSNWCQDNIISLMSAGILGVPPASGALSTQTSTASMVQPPQGDVEAMYPALPPYSNCG
DLYSEPVSFHDPQGNPGLAYSPQDYQSAKPALDSNLFPMIPDYNLYHHPNDMGSIPEHKPFQGMDPIRVNPPPITP
LETIKAFKDKQIHPGFGSLPQPPLTLKPIRPRKYPNRPSKTPLHERPHACPAEGCDRRFSRSDELTRHLRIHTGHKPFQ
CRICMRSFSRSDHLTTHIRTHTGEKPFACEFCGRKFARSDERKRHAKIHLKQKEKKAEKGGAPSASSAPPVSLAPVVT
TCA
SEQ ID NO: 2 is the nucleotide acid sequence of the human *EGR3* transcript: 
ATGACCGGCAAACTCGCCGAGAAGCTGCCGGTGACCATGAGCAGTTTGCTAAACCAACTGCCTGACAATCTGT
ACCCCGAGGAGATCCCCAGCGCGCTCAACCTCTTCTCCGGCAGCAGCGACTCGGTAGTCCATTACAATCAGAT
GGCTACAGAGAATGTAATGGACATCGGTCTGACCAACGAGAAGCCCAACCCGGAACTCTCTTACTCCGGCTCC
TTCCAGCCAGCCCCCGGCAACAAGACCGTGACCTACTTGGGAAAGTTCGCCTTCGACTCCCCTTCCAACTGGTG
CCAGGACAACATCATTAGCCTCATGAGCGCCGGCATCTTGGGGGTGCCCCCGGCTTCAGGGGCGCTCAGCACG
CAGACGTCCACGGCCAGCATGGTGCAGCCACCGCAGGGTGACGTGGAGGCCATGTATCCCGCGCTACCCCCCT
ACTCCAACTGCGGCGACCTCTACTCAGAGCCCGTGTCTTTCCACGACCCCCAGGGCAATCCCGGGCTCGCCTAT
TCCCCCCAGGATTACCAATCGGCCAAGCCGGCGTTGGACAGCAATCTCTTCCCCATGATTCCTGACTACAACCT
CTACCACCACCCCAACGACATGGGCTCCATTCCGGAGCACAAGCCCTTCCAGGGCATGGACCCCATCCGGGTC
AACCCGCCCCCTATTACCCCTCTGGAGACCATCAAGGCATTCAAAGACAAGCAGATCCACCCGGGCTTTGGCA
GCCTGCCCCAGCCGCCGCTCACCCTCAAGCCCATCCGGCCCCGCAAGTACCCCAACCGGCCTAGCAAGACACC
GCTCCACGAACGGCCCCACGCGTGCCCGGCCGAGGGCTGCGACCGCCGTTTCAGCCGTTCGGACGAGCTGAC
CCGGCACCTGCGCATCCACACGGGCCACAAGCCCTTCCAGTGCCGGATCTGCATGCGGAGCTTCAGCCGCAGC
GACCACCTCACCACTCACATCCGCACTCATACGGGCGAGAAGCCCTTTGCCTGCGAGTTCTGCGGGCGCAAGT
TTGCGCGCAGCGACGAGCGCAAGCGCCACGCCAAGATCCACCTCAAGCAAAAGGAGAAGAAGGCGGAGAAG
GGCGGTGCACCCTCTGCATCCTCGGCGCCCCCCGTGTCGCTGGCCCCCGTGGTCACCACCTGCGCCTGA
SEQ ID NO: 3 is the amnio acid sequence Zebrafish Egr3 protein: 
MTGKLADKIPITMSSLINTIPDSLYPEEDIPTSMNIFANPDSISHYSQMNTDNIMDLGMGGEKGSGEIQYGSGSFQS
GRSGQTVTYLGKFAFDTPPSGSIGGSGWCPDNNIISLVSAGILGVSPSPGNITTQTSSSSGSMGGQSSDMEQVYAPP
LPAYSTCGEIYPEQVTFHHSPATSSSLPYPSSDYHTTSKPGMDGSLFSMIPDYNLFHHQGDVGVMEHKPFQTMDPI
 
RVNPPPITPLETIRAFKDKQQIHPGFLGGQQHVSQHHQPSQTLALKPIRPRKYPNRPSKTPVHERPHACPAENCDRR
FSRSDELTRHLRIHTGHKPFQCRICMRSFSRSDHLTTHIRTHTGEKPFSCEFCGRKFARSDERKRHAKVHLKQKDKKP
TDKGSSAGAGGNHTSPPSSCGSAGPSSTNIMTVTTCA
SEQ ID NO: 4 is the nucleotide sequence of the Zebrafish *egr3* transcript:

ATGACAGGTAAACTAGCGGACAAGATCCCTATTACCATGAGCAGTTTAATCAACACCATACCTGACAGTCTCTA
CCCAGAAGAAGACATCCCAACTTCTATGAACATTTTCGCAAACCCGGACTCCATTTCGCACTACTCGCAGATGA
ATACAGATAATATCATGGACTTGGGGATGGGCGGCGAGAAGGGCAGCGGAGAAATTCAGTACGGCTCCGGC
AGCTTCCAGTCTGGGCGAAGTGGGCAGACCGTCACCTACCTGGGCAAGTTTGCATTCGACACCCCTCCTTCAG
GCAGCATCGGCGGCTCCGGCTGGTGTCCCGACAACAACATCATAAGCCTGGTGAGTGCAGGCATCCTAGGGG
TGTCCCCTTCACCTGGAAACATCACCACCCAGACGTCTTCATCCAGCGGCAGCATGGGTGGCCAGTCTTCAGAC
ATGGAGCAAGTATACGCACCACCCCTACCTGCCTACTCCACCTGTGGAGAAATATACCCAGAGCAGGTGACGT
TCCACCACAGTCCGGCCACGTCCTCATCCTTGCCCTACCCGAGCTCTGACTACCACACCACCTCTAAACCAGGCA
TGGACGGCAGTCTCTTCTCCATGATCCCAGACTACAACCTCTTCCATCATCAGGGAGACGTAGGGGTGATGGA
GCATAAACCCTTTCAAACCATGGACCCTATCAGGGTGAACCCGCCTCCCATAACTCCCCTCGAGACCATTCGAG
CTTTTAAGGATAAGCAGCAGATTCATCCCGGCTTCCTCGGTGGGCAACAGCACGTTTCGCAACATCATCAACCC
TCTCAGACACTAGCTCTCAAACCTATCCGTCCAAGAAAGTATCCCAACAGGCCGAGCAAAACTCCAGTGCACGA
GAGACCACATGCCTGTCCGGCCGAGAACTGTGACCGGCGTTTTTCGCGATCCGATGAGTTGACGCGGCACCTG
CGCATCCACACAGGACACAAACCCTTCCAGTGCCGCATCTGCATGCGGTCCTTCAGTCGCAGTGACCATCTCAC
CACACACATCCGCACACACACCGGCGAGAAGCCGTTCTCTTGCGAATTCTGTGGACGCAAGTTTGCGCGGAGC
GACGAGCGAAAGCGGCACGCCAAGGTGCACCTCAAACAGAAGGACAAGAAACCCACGGACAAGGGCAGCAG
TGCTGGGGCAGGGGGGAACCACACTTCTCCTCCCAGCTCCTGTGGGAGCGCAGGACCCAGCAGTACTAACATC
ATGACCGTCACCACTTGTGCATGA

The human and Zebrafish *EGR3* transcripts with the 5' and 3'-UTRs are shown in SEQ ID NOs 5 and 6.

The sequence identity of at least 80%, preferably at least 90% and most preferably at least 95% Means and methods for determining sequence identity are known in the art. Preferably, the BLAST (Basic Local Alignment Search Tool; Protein BLAST and Nucleotide BLAST) program is used for determining the sequence identity with regard to the sequences are described herein. Also envisioned herein are with more increasing preference sequence identities of least 96%, at least 97% and at least 98%, at least 99% and 100%.

An expression vector may be a plasmid that is used to introduce a specific transcript into a target cell. Once the expression vector is inside the cell, the protein that is encoded by the gene is produced by the cellular-transcription and translation machinery ribosomal complexes. The plasmid is in general engineered to contain regulatory sequences that act as enhancer and/or promoter regions and lead to efficient transcription of the transcript. In accordance with the present invention the expression vector preferably contains an endocardial-specific promoter and/or valve-cell promoter. This ensures that the nucleic acid sequence is only expressed in the heart, in particular valve and may avoid potential unwanted side effects by expression in other organs and/or cell-types.

Non-limiting examples of expression vectors include prokaryotic plasmid vectors, such as the pUC-series, pBluescript (Stratagene), the pET-series of expression vectors (Novagen) or pCRTOPO (Invitrogen) and vectors compatible with an expression in mammalian cells like pSBbi (Plasmid #60511), pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, plZD35, pLX!N, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Intvitrogen). For the formulation of a pharmaceutical composition a suitable vector is selected in accordance with good manufacturing practice. Such vectors are known in the art, for example, from Ausubel et al, Hum Gene Ther. 2011 Apr; 22(4):489-97 or Allay et al., Hum Gene Ther. May 2011; 22(5): 595-604.

A typical mammalian expression vector contains the promoter element, which mediates the initiation of transcription of mRNA, the protein coding sequence, and signals required for the termination of transcription and polyadenylation of the transcript. Moreover, elements such as origin of replication, drug resistance gene, regulators (as part of an inducible promoter) may also be included. The *lac* promoter is a typical inducible promoter, useful for prokaryotic cells, which can be induced using the lactose analogue *isopropylthiol-b-D-galactoside.* ("IPTG"). For recombinant expression and secretion, the polynucleotide of interest may be ligated between e.g. the PelB leader signal, which directs the recombinant protein in the periplasm and the gene III in a phagemid called pHEN4 (described in Ghahroudi et al, 1997, FEBS Letters 414:521-526). Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG, pRSVcat (ATCC 37152), pSV2dhfr (ATCC 37146) and pBC12MI (ATCC 67109). Alternatively, the recombinant (poly)peptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al.1991, Biochem J. 227:277-279; Bebbington et al. 1992, Bio/Technology 10:169-175). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria. For vector modification techniques, see Sambrook and Russel (2001), Molecular Cloning: A Laboratory Manual, 3 Vol.. Generally, vectors can contain one or more origins of replication (ori) and inheritance systems for cloning or expression, one or more markers for selection in the host, e.g., antibiotic resistance, and one or more expression cassettes. Suitable origins of replication (ori) include, for example, the Col E1, the SV40 viral and the M 13 origins of replication.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e.g., translation initiation codon, promoters, enhancers, and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers, and/or naturally-associated or heterologous promoter regions. Preferably, the nucleotide sequence as defined in item (a) of the above preferred embodiment of the invention is operatively linked to such expression control sequences allowing expression in prokaryotic or eukaryotic cells.

The host may be a prokaryotic or eukaryotic cell. A suitable eukaryotic host may be a mammalian cell, an amphibian cell, a fish cell, an insect cell, a fungal cell or a plant cell. Representative examples of bacterial cells are *E. coli,* Streptomyces and *Salmonella typhimurium* cells; of fungal cells are yeast cells; and of insect cells are Drosophila S2 and Spodoptera Sf9 cells. It is preferred that the cell is a mammalian cell such as a human cell. Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. The cell may be a part of a cell line, preferably a human cell line. Appropriate culture mediums and conditions for the above-described host cells are known in the art. The host is preferably a host cell and more preferably an isolated host cell. The host is also preferably a non-human host.

In accordance with a further preferred embodiment of the second aspect the subject having a valve defect has a congenital valve disease, preferably selected from bicuspid aortic valve, pulmonary atresia, mitral valve prolapse, pulmonary valve stenosis, or the subject having a valve defect has an acquired valve disease selected from rheumatic heart disease, mitral valve regurgitation, aortic regurgitation, tricuspid regurgitation, and infective endocarditis.

The above diseases are non-limiting but preferred examples of valve diseases that are associated with valve defects. As discussed, congenital valve diseases, appear during early development and affect the structural integrity and functionality of heart valves. On the other hand, acquired valve diseases develop during age or as unwanted side effect of medicaments.

The present invention relates in a third aspect to a non-human animal model for a valve defect, wherein in the non-human animal the expression of *EGR3* is knocked-out or knocked-down.

It is shown in the appended examples that when the expression of *EGR3* is knocked-out or knocked-down in an animal, then the animal has defective valves: Thus, such non-human animals can be used as models for a valve defect. For instance, new drugs and/or surgical methods for the treatment of valve defects can be tested with such non-human animals

The non-human animal is preferably a vertebrate, more preferably a mammal and more preferably a mammal selected from cat, dog, horse, cattle, swine, goat, sheep, mouse, rat, monkey. Among non-mammalian vertebrates zebrafish is preferred.

The *EGR3* is preferably knocked-out via CRISPR-Cas technology. The *EGR3* is preferably knocked-down via RNA interference, for, example; antisense nucleic acid molecule (e.g. antisense oligonucleotide), such as siRNA, shRNA, morpholinos or a Gapmer.

The *EGR3* can also be knocked-out via Cre-lox recombination. The system consists of a single enzyme, Cre recombinase, that recombines a pair of short target sequences called the Lox sequences. This system can be implemented without inserting any extra supporting proteins or sequences. The Cre enzyme and the original Lox site called the *LoxP* sequence are derived from bacteriophage P1.

The figures show.

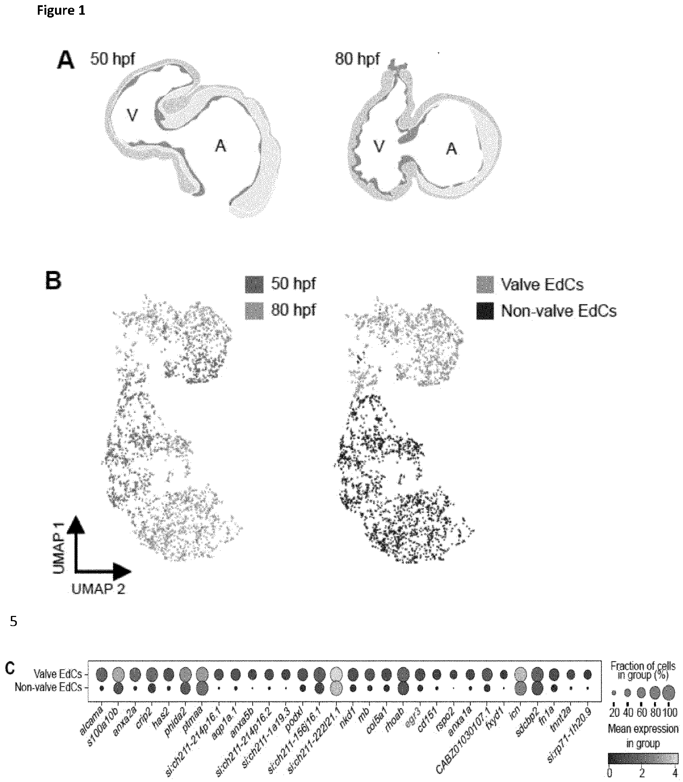

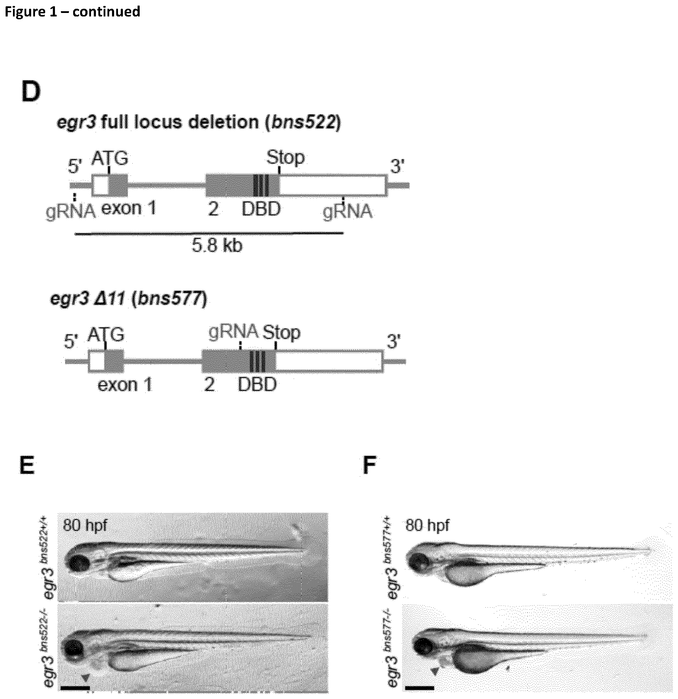

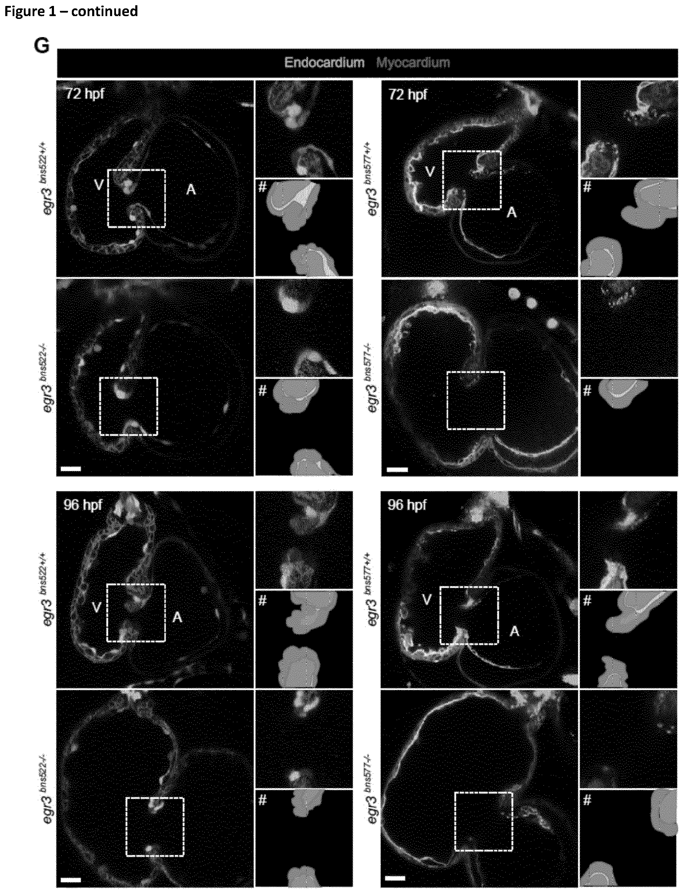

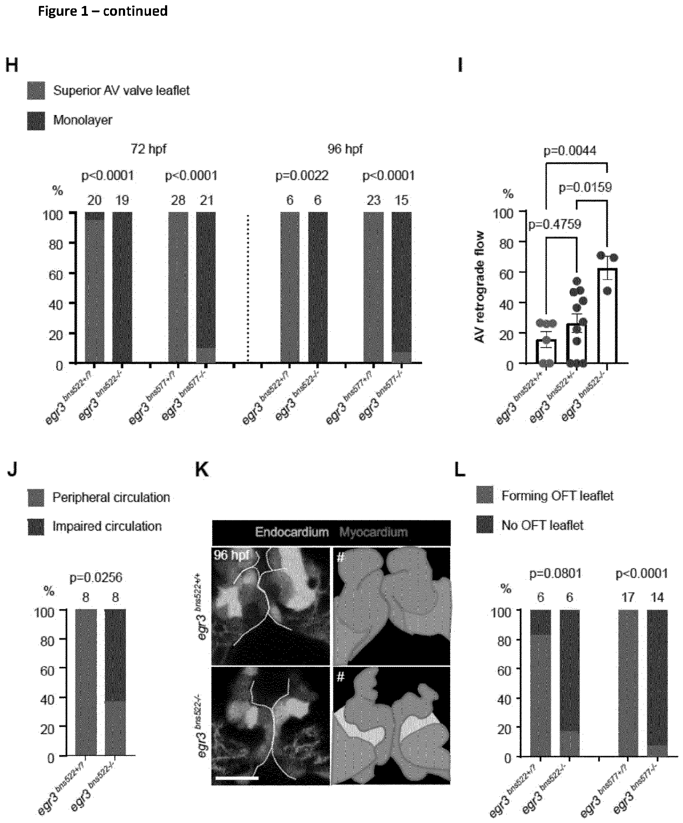

**Fig. 1****. Egr3 is required for cardiac valve formation.** (A), Schematic of hearts isolated for scRNA-seq; valve endocardial cells (magenta), non-valve endocardial cells (blue). (B), scRNA-seq of endocardial cells from 50 and 80 hpf dissected zebrafish hearts. (C), Dot plot of 30 most differentially expressed genes in valve compared with non-valve endocardial cells. (D), Schematic of generation of *egr3* full locus deletion (*bn1022*) and Δ11 (*bn1077*) alleles. (E-F), Brightfield images of *egr3*^{*+*/*+*} and *egr3⁻¹⁻* sibling larvae at 80 hpf; red arrowheads point to pericardial edema. (G), Confocal images of representative hearts from 72 and 96 hpf *egr3*^{*+*/*+*} and *egr3*^{*-*/*-*} sibling larvae. (H), Percentage of *egr3*^{*+*/*+*} and *egr3*^{*-*/*-*} sibling larvae with a superior valve leaflet and without a superior valve leaflet (i.e., endocardial monolayer) at 72 and 96 hpf; 7 and 4 independent experiments, respectively. (I), AV retrograde blood flow shown as a fraction of three cardiac cycles; n = 6, 11, and 3; 1 experiment; mean ± SEM, one-way ANOVA followed by Tukey's post hoc test. (J), Peripheral circulation in the caudal vein plexus at 80 hpf, 1 experiment. (K), Confocal images of representative outflow tract valves from 96 hpf *egr3*^{+/+} and *egr3*^{-/-} sibling larvae. (L), Percentage of *egr3*^{*+*/*+*} and *egr3*^{*-*/*-*} sibling larvae with and without forming outflow tract leaflets at 96 hpf; 4 independent experiments. (G, K), Endocardial cells are marked by *Tg(kdrl:eGFP)* expression (cyan), myocardial cells by *Tg*(*myl*7: BFP-CAAX) expression (magenta). # indicates illustrative vectorized cartoons of the valves. (H, J, L), Fisher's exact test. AV = atrioventricular, V = ventricle, A = atrium, OFT = outflow tract, DBD = DNA binding domain, EdC = endocardial cell. Scale bars (E, F) = 400 µm, (G, K) = 20 µm.

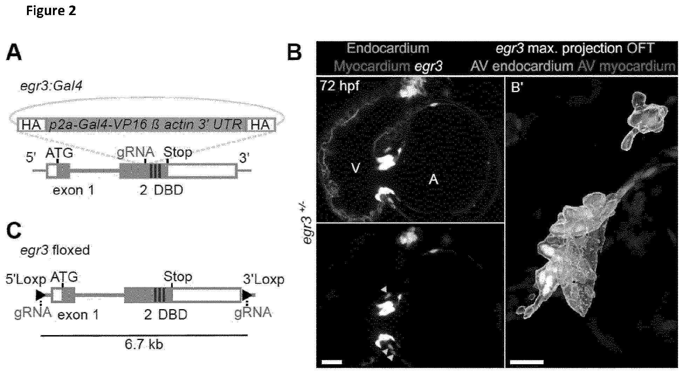

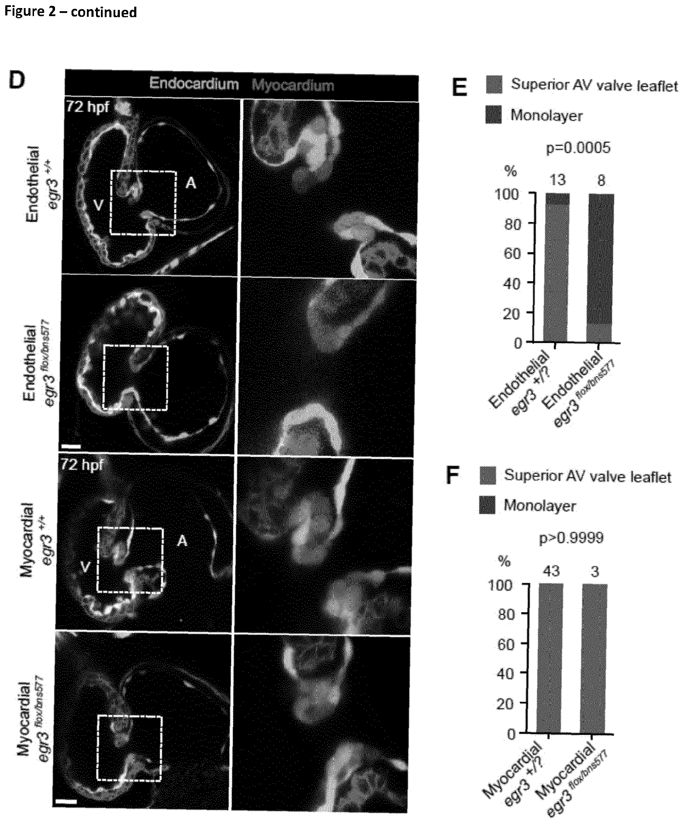

**Fig. 2****. Endothelial-specific deletion of *egr3* recapitulates the global mutant phenotype.** (A), Schematic of the knock-in reporter (*Pt*(*egr3:Gal4-VP16*)) generated by inserting *Gal4-VP16* in exon 2 of *egr3.* (B), *Pt*(*egr3*:Gal4-VP16); *Tg*(*UAS*:eGFP) expression (grey) at 72 hpf; endocardial cells are marked by *Tg*(*kdrl*:Hsa.HRAS-mCherry) expression (cyan) and myocardial cells by *Tg*(*myl*7:BFP-CAAX) expression (magenta); yellow arrowheads point to AV canal *egr3* reporter+ cardiomyocytes; (B') maximum projection of *Pt*(*egr3*:Gal4-VP16); *Tg*(*UAS*:eGFP) expression with image segmentation of *egr3* reporter⁺ AV canal endocardial cells (cyan), *egr3* reporter⁺ AV canal myocardial cells (magenta), and *egr3* reporter⁺ outflow tract cells (yellow). (C), Schematic of *egr3* floxed allele *Pt(egr3:loxP-egr3-loxP*). (D), Confocal images of representative hearts from 72 hpf *egr3*^{*+*/}*⁺,* endothelial cell-specific *egr3*^{*-*/-}, and myocardial cell-specific *egr3*^{*-*/*-*} larvae using the *egr3* floxed allele line. (E-F), Percentage of 72 hpf *egr3*^{*+*/}*^{?},* endothelial cell-specific *egr3*^{*-*/*-*} (E), and myocardial cell-specific *egr3*^{*-*/*-*} (F) larvae with a superior valve leaflet and without a superior valve leaflet (i.e., endocardial monolayer); 1 and 2 independent experiments, respectively; Fisher's exact test. AV = atrioventricular, V = ventricle, A = atrium. Scale bars = 20 µm.

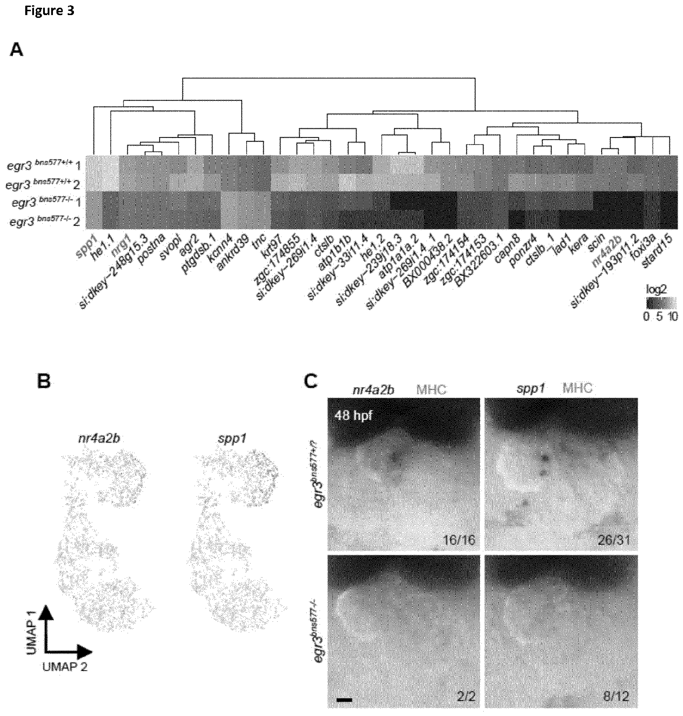

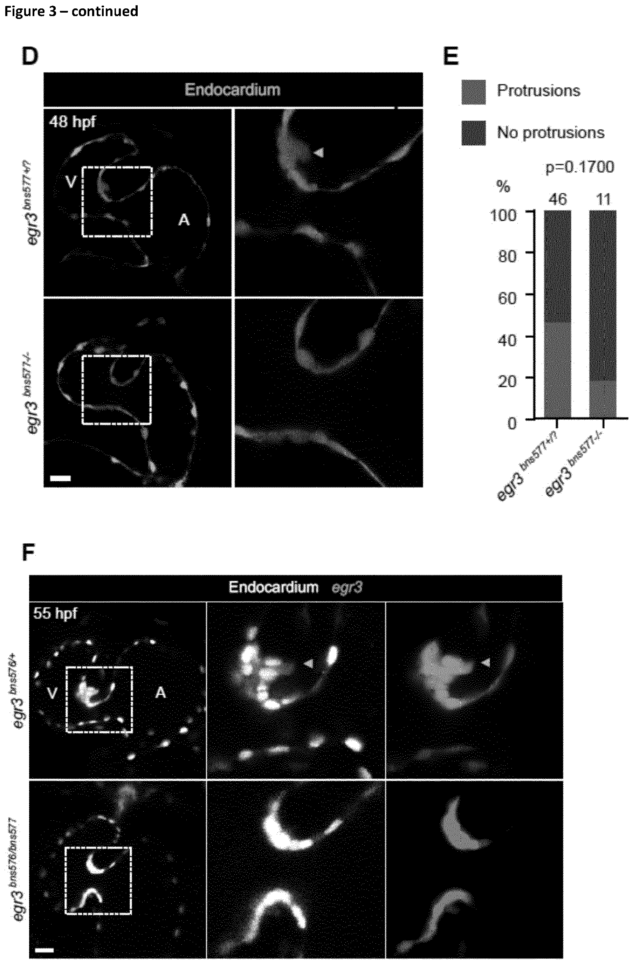

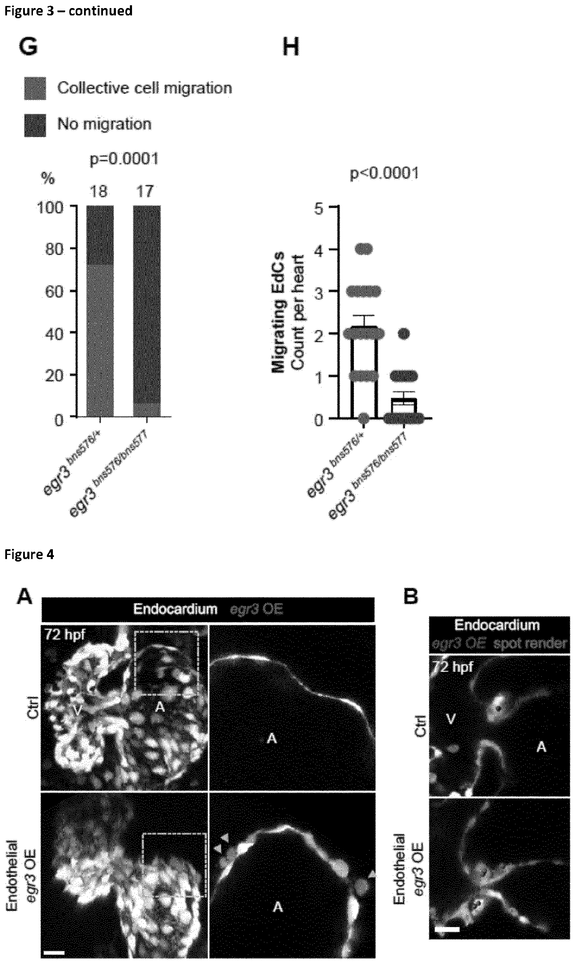

**Fig. 3****. *egr3* is necessary for endocardial cell migration, which is required for cardiac valve formation.** (A), RNA-seq heatmap analysis of differentially expressed genes; fold-change ± 1.5, padj < 0.05, mean > 5. (B), scRNA-seq endocardial expression pattern of Egr3 targets *nr4a2b* and *spp1.* (C), *in situ* hybridization for Egr3 targets *nr4a2b* and *spp1* in *egr3*^{*+*/*+*} and *egr3*^{*-*/*-*} siblings at 48 hpf. (D), Confocal images of representative hearts from 48 hpf *egr3*^{*+*/}*^{?} and egr3*^{*-*/*-*} sibling embryos displaying, respectively, AV endocardial cell protrusions towards the ECM and no protrusions; endocardial cells are marked by *Tg(kdrl:eGFP)* expression (cyan). (E), Percentage of hearts from 48 hpf *egr3*^{*+*/*?*} and *egr3*^{*-*/*-*} sibling embryos displaying AV endocardial cell protrusions towards the ECM; 3 independent experiments. (F), Confocal images of representative hearts from 55 hpf *egr3*^{*+*/*-*} and *egr3*^{*-*/*-*} sibling embryos displaying, respectively, collective AV endocardial cell migration and no migration; endocardial cells are marked by *Tg*(*kdrl*:NLS-mCherry) expression (grey) and AV endocardial cells by *Pt(egr3:Gal4-*VP16);*Tg*(*UAS*:eGFP) expression (green). (G), Percentage of hearts from 55 hpf *egr*3^{+/-} and *egr*3^{-/-} sibling embryos displaying AV endocardial cell migration; 3 independent experiments. (H), Number of migrating AV endocardial cells per heart in 55 hpf *egr3*^{*+*/*-*} and *egr3*^{*-*/*-*} sibling embryos; n = 18 and 17 embryos; 3 independent experiments. Student's t test, mean ± SEM. (E, G), Fisher's exact test. AV = atrioventricular, V = ventricle, A = atrium. Scale bars = 20 µm.

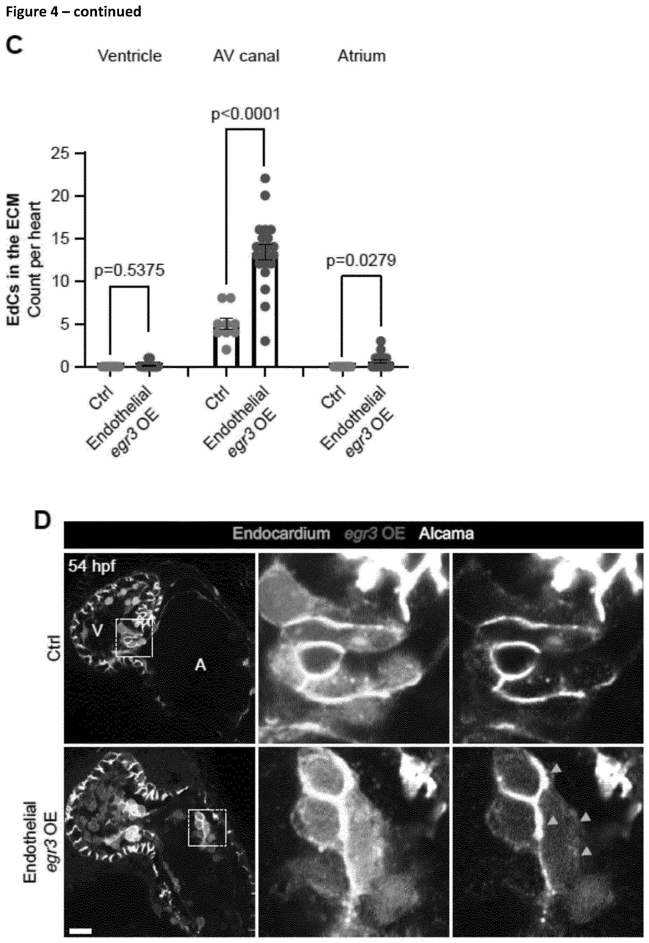

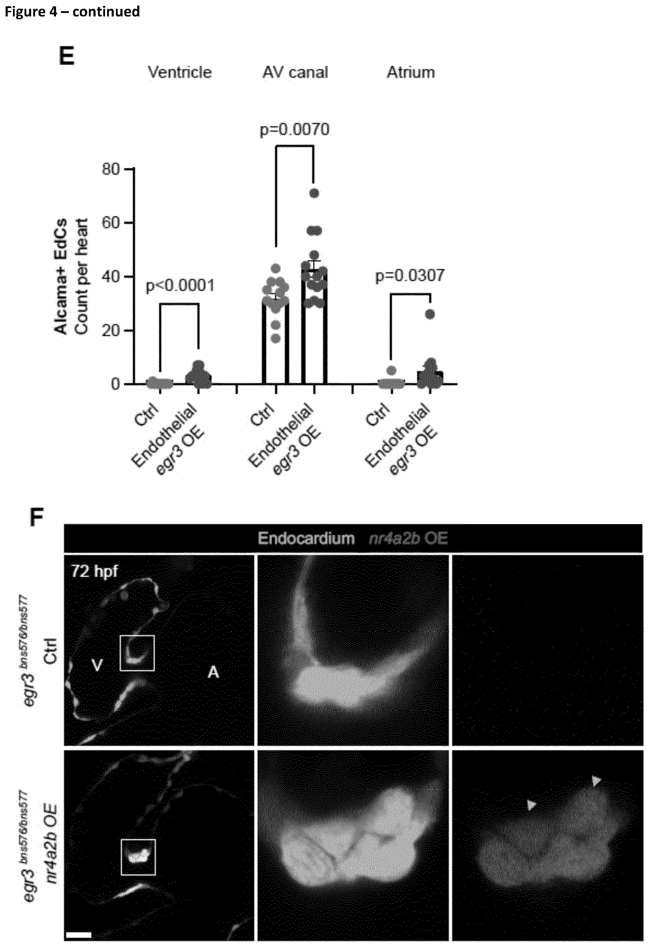

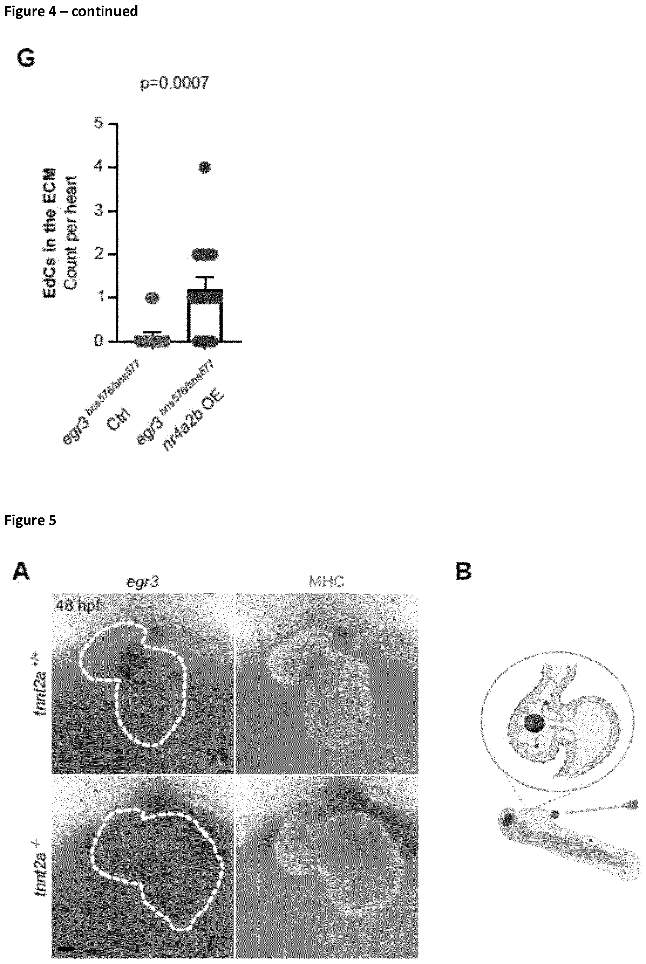

**Fig. 4****. *egr3* triggers endocardial cell migration and Alcama expression.** (A), 3D reconstruction of representative hearts from control *(Tg(flila:Gal4);Tg(UAS:Kaede))* and endothelial-specific *egr3* overexpressing *(Tg(flila:Gal4);Tg(UAS:Kaede);Tg(UAS:egr3-p2a-dTomato))* 72 hpf larvae; magnified regions show single z-planes of the atrium from control and endothelial-specific *egr3* overexpressing 72 hpf larvae; yellow arrowheads point to *Tg*(*UAS*:egr3-p2a-dTomato)⁺ atrial endocardial cells in the adjacent ECM. (B), Confocal images of representative AV VIC quantification through the spot render function in Imaris; VICs (magenta spots). (C), Quantification of endocardial cells present in the ECM per cardiac region in 72 hpf endothelial-specific *egr3* overexpressing larvae; n = 9 control and 22 *egr3 OE* larvae, n = 0 and 0.14 EdCs (ventricle), n = 5 and 13.36 EdCs (AV canal), n = 0 and 0.59 EdCs (Atrium). (D), Confocal images of representative hearts from control (*Tg(flila:Gal4);Tg(UAS:Kaede*)) and endothelial-specific *egr3* overexpressing (*Tg(flila:Gal4);Tg(UAS:Kaede);Tg(UAS:egr3-p2a-dTomato))* 54 hpf embryos immunostained for Alcama; magnified regions show Alcama⁺ cells in the AV canal of the control heart and in the atrium of the *Tg(UAS:egr3-p2a-dTomato)* heart. (E), Quantification of Alcama⁺ endocardial cells per cardiac region of control and endothelial-specific *egr3* overexpressing embryos at 54 hpf; n = 13 control and 14 *egr3 OE* embryos, n = 0.07 and 3.28 EdCs (ventricle), n = 31.77 and 42.86 EdCs (AV canal), n = 0.38 and 4.71 EdCs (Atrium); 3 independent experiments. (F), Confocal images of representative hearts from control *Pt(egr3:Gal4-VP16)* and egr3-driven *nr4a2b* overexpressing *(Pt(egr3:Gol4-VP16); Tg(UAS:nr4a2b-p2a-dTomato)) egr3*^{*-*/*-*} larvae at 72 hpf. (G), Quantification of endocardial cells present in the AV canal ECM of control and *nr4a2b* overexpressing *egr3*^{*-*/*-*} larvae at 72 hpf; n = 16 and 15 larvae; 2 independent experiments. (C, E, G), Student's t test, mean ± SEM. AV = atrioventricular, V = ventricle, A = atrium, ECM = extracellular matrix, VIC = valve interstitial cell. Scale bars = 20 µm.

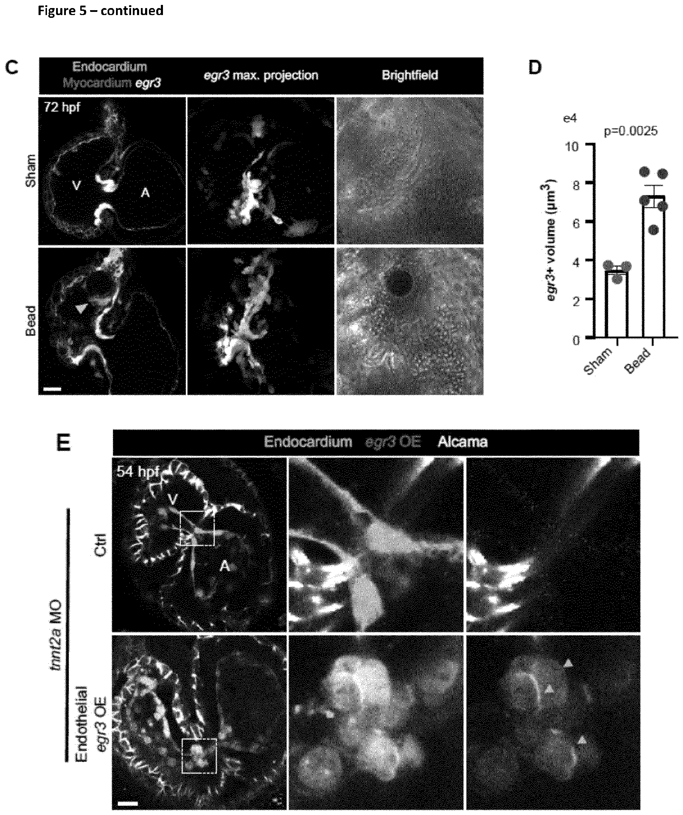

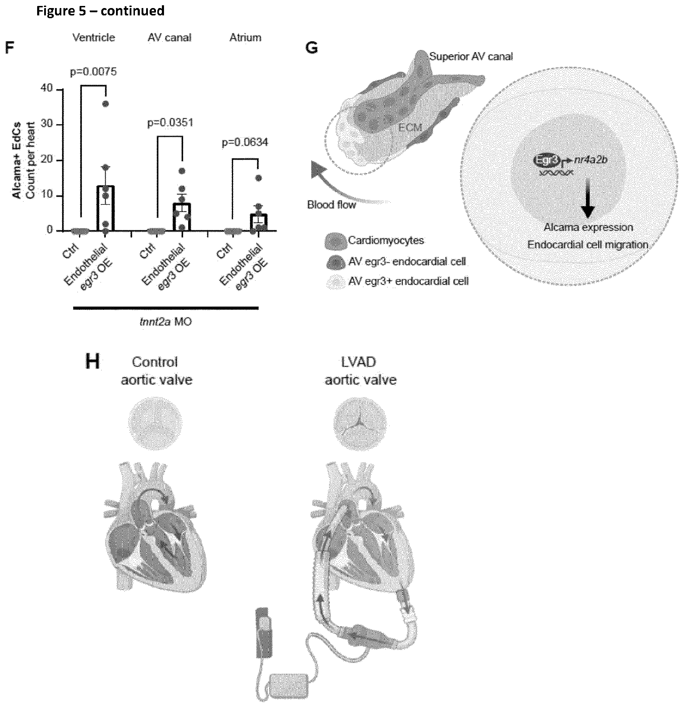

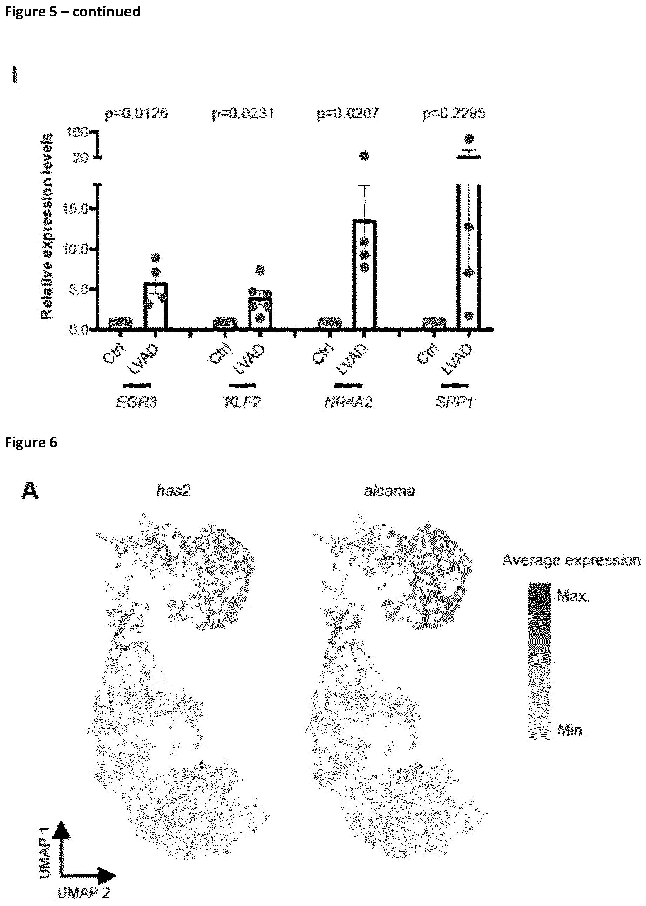

**Fig. 5****. *egr3* expression in the heart is regulated by biomechanical forces.** (A), *in situ* hybridization for *egr3* expression in 48 hpf *tnnt2a*^{*+*/*+*} and *tnnt2a*^{*-*/*-*} sibling embryos. (B), Illustration of bead insertion into a 48 hpf heart. (C), *Pt*(*egr3*:Gal4-VP16); *Tg*(*UAS*:eGFP) expression (grey) at 72 hpf in bead-inserted larva vs sham; myocardial cells are marked by *Tg*(*myl7*:BFP-CAAX) expression (magenta) and endocardial cells by *Tg*(*kdrl*:Hsa.HRAS-mCherry) expression (cyan). Red dotted circle highlights inserted bead; yellow arrowhead points to *egr3⁺* cells near the bead. (D), Quantification of total volume of *egr3* reporter⁺ cells in bead-inserted larvae and sham; n = 3 sham and 5 experimental larvae; 1 experiment. (E), Confocal images of representative hearts from control *(Tg(fli1a:Gal4);Tg(UAS:Kaede))* and endothelial-specific *egr3* overexpressing *(Tg(fli1a:Gal4);Tg(UAS:Kaede);Tg(UAS:egr3-p2a-dTomato)) tnnt2a* MO injected embryos immunostained for Alcama at 54 hpf; magnified regions show the AV canal of *tnnt2a* MO injected embryos with rescued Alcama expression in the *Tg(UAS:egr3-p2a-dTomato)* embryo. (F), Quantification of Alcama⁺ endocardial cells per cardiac region of *tnnt2a* MO injected embryos at 54 hpf; n = 6 control and 6 *egr3 OE* embryos, n = 0.00 and 8.00 EdCs (ventricle), n = 0.00 and 13.00 EdCs (AV canal), n = 0.00 and 4.83 EdCs (Atrium); 2 independent experiments. (G), Oscillatory shear stress induces the expression of the mechanosensitive transcription factor gene *egr3* in valve EdCs where it orchestrates valvulogenesis by promoting Alcama expression and their migration via Nr4a2b. (H), Schematic of aortic valve collection from LVAD patients and control donors. (I), Relative mRNA levels of *EGR3, KLF2, NR4A2,* and *SPP1* from aortic valves of LVAD patients and control donors; n = 4 and 4-6; 1 experiment. (D, F, I), Student's t test, mean ± SEM. (B, H), Illustrations were generated with Biorender.com. V = ventricle, A = atrium. Scale bars = 20 µm.

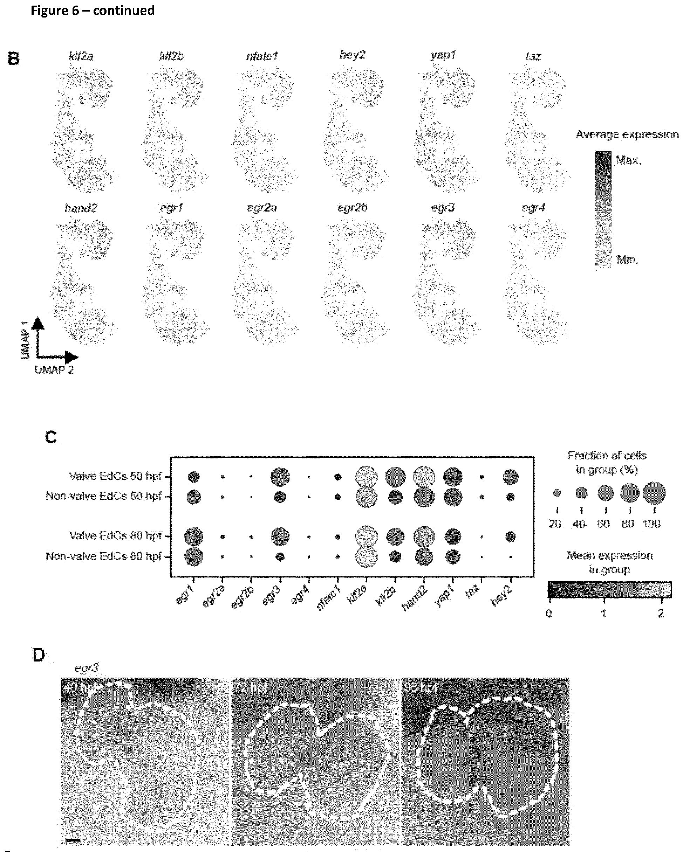

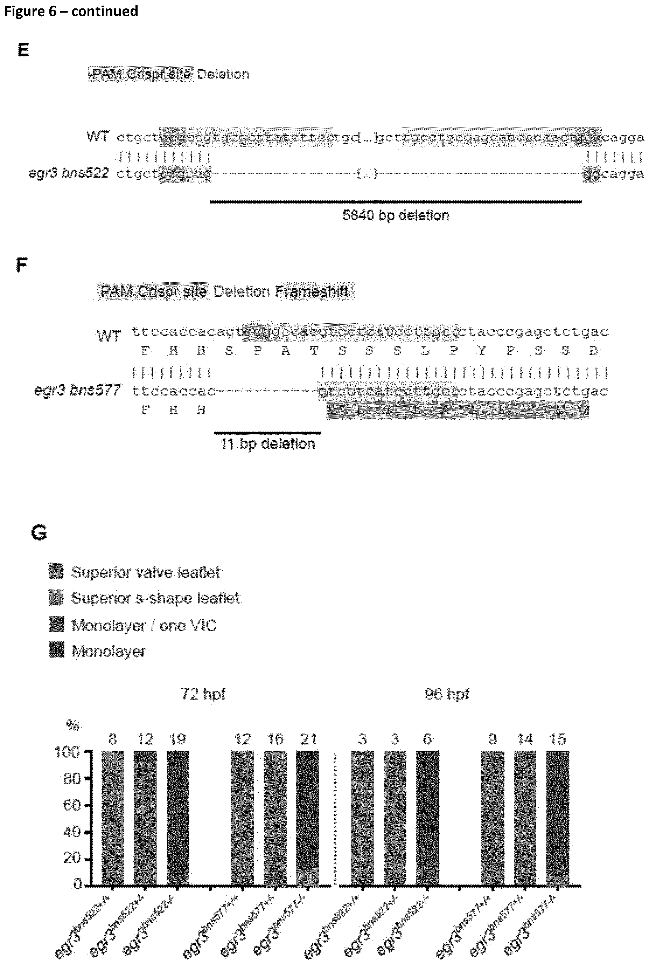

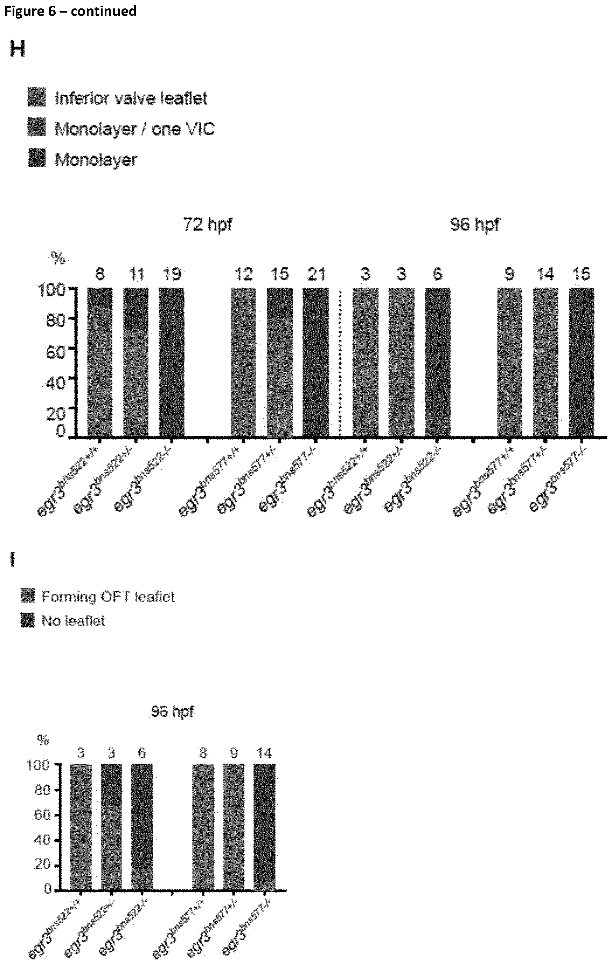

**Fig. 6****. *egr3* expression is enriched in valve endocardial cells.** (**A**), scRNA-seq endocardial average expression pattern of valve markers *ha7* and *alcama.* (**B**), Endocardial expression pattern of key transcription factor genes; *egr3* expression is enriched in the valve endocardium. (**C**), Dot plot of key transcription factor genes in valve and non-valve endocardial cells at 50 and 80 hpf. (**D**), *in situ* hybridization for *egr3* expression at 48, 72 and 96 hpf. (**E-F**), Schematic of *egr3* full locus deletion (*bn1022*) and Δ11 (*bn1077*) mutant alleles. (**G-I**), Percentage of larvae without or with a superior (**G**), inferior (**H**), and OFT (**I**), valve leaflet phenotype at 72 and 96 hpf; 7 and 4 independent experiments, respectively, (related to Fig. 1H, L). Scale bar = 20 µm.

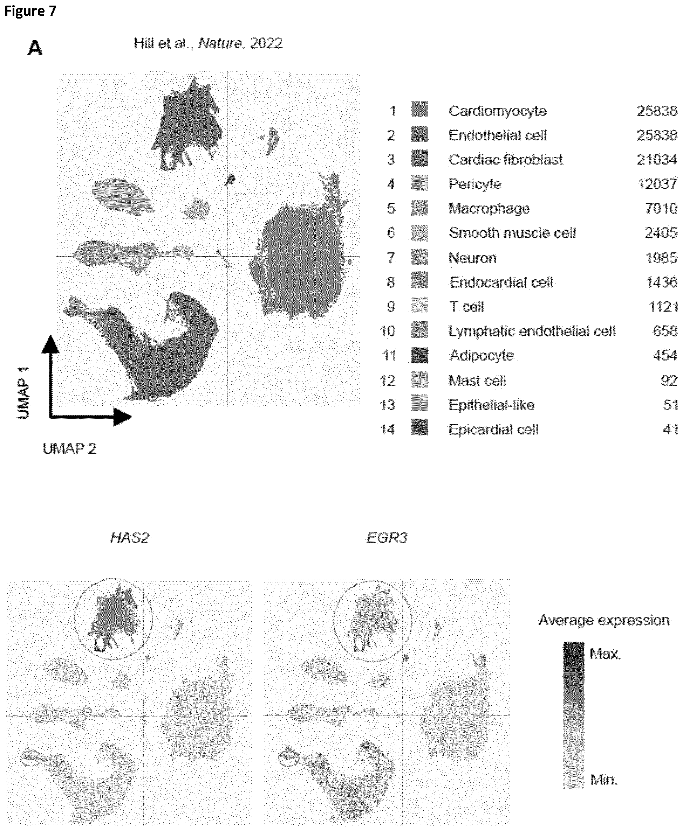

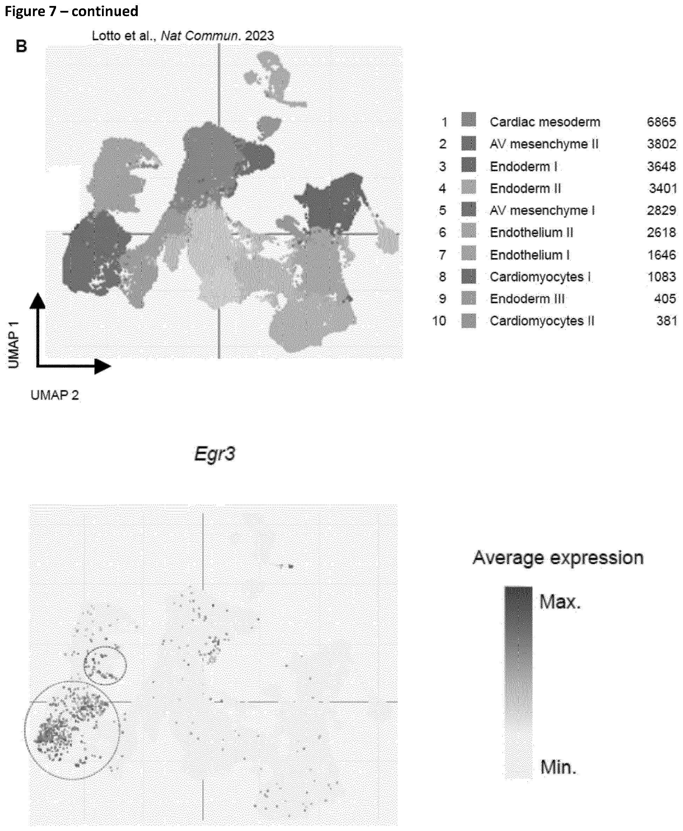

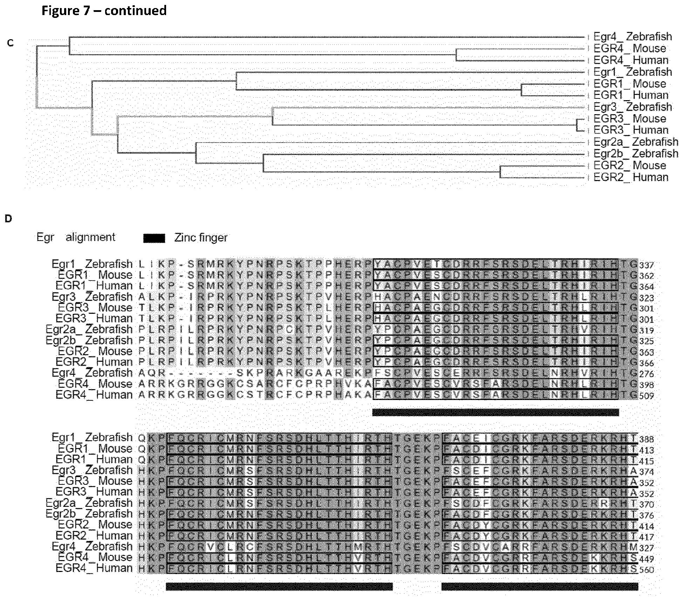

**Fig. 7****. *EGR3* cardiac valve expression is conserved across species.** (**A**), scRNA-seq of adult human heart (41). *EGR3* is expressed in cardiac valve populations of endocardial cells (blue circle) and cardiac fibroblasts (red circle). (**B**), scRNA-seq of mouse heart cells at E7.75, 8.25, 9.25, 10.5, and 12.5 (42). *Egr3* is expressed in the AV mesenchyme (red circle) and endocardial (blue circle) populations. (**C**), Phylogenetic tree of human, mouse, and zebrafish Egr family members. (**D**), Protein alignment of zinc finger DNA binding domain of human, mouse, and zebrafish Egr family members. AV = atrioventricular.
**Fig. 8****. Cell-specific role of Egr3.** (A), *Pt*(egr3:Gal4-VP16); *Tg*(*UAS*:eGFP*)* expression in the whole embryo at 55 hpf, and in the heart at 34, 48 and 72 hpf. (B), Confocal images of representative hearts from 72 hpf *egr3*^{*bn1076*/*+*} and *egr3*^{*bn1076*/*bn1022*} sibling larvae. (C), Percentage of *egr3* ^{*+*/*-*} and *egr3*^{*-*/*-*} sibling larvae with a superior valve leaflet and without a superior valve leaflet (i.e., endocardial monolayer) at 72 hpf; 1 experiment. Fisher's exact test. (D), Schematic of *egr3* floxed allele recombination. (E), Brightfield images of *cre* mRNA injected 72 hpf *egr3*^{*+*/*+*} and *egr3*^{*flox*/*bn1077*} sibling larvae. (F), Confocal images of representative *cre* mRNA injected 72 hpf *egr3*^{*+*/*+*} and *egr3*^{*flox*/*bn1077*} sibling larvae; endocardial cells are marked by *Tg*(*kdrl*:Hsa.HRAS-mCherry) expression (cyan). (G-H), Brightfield images of *egr3*^{*+*/}*⁺,* endothelial-specific *egr3*^{*-*/*-*} (G), and myocardial-specific *egr3*^{*-*/*-*} (H), sibling larvae at 72 and 96 hpf, respectively. (I) Percentage of larvae with or without a superior AV valve leaflet (i.e., endocardial monolayer) at 72 hpf; 1 and 2 independent experiments (related to Fig. 2E, F). AV = atrioventricular, V = ventricle, A = atrium. Scale bars (A (whole embryo), E, G, H) = 400 µm, (A (hearts), B, F) = 20 µm.

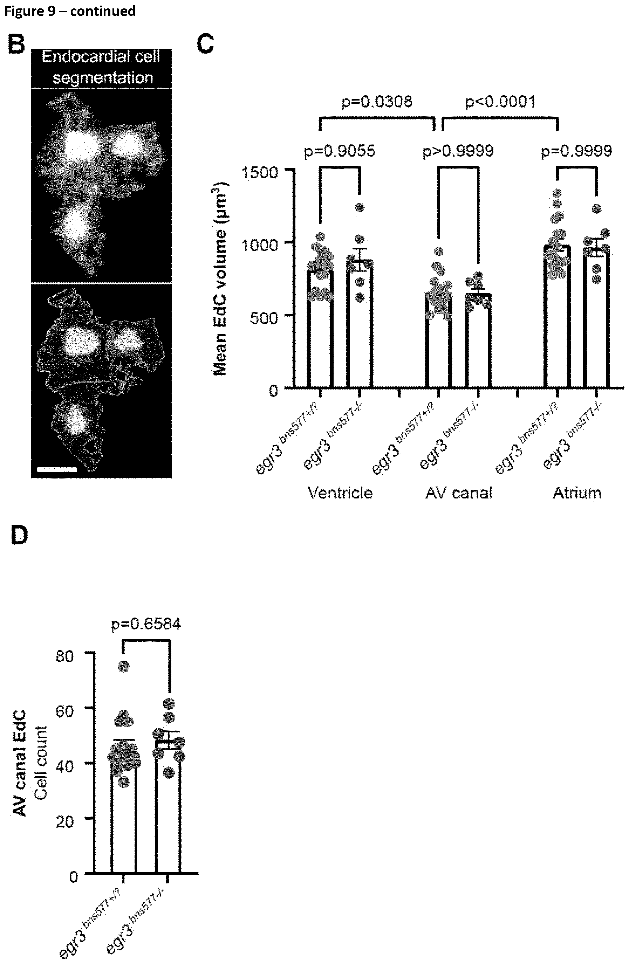

**Fig. 9****. Endocardial cell morphology is not altered in *egr3* mutants at the onset of the atrioventricular valve phenotype.** (**A**), 3D reconstruction of a zebrafish heart at 48 hpf. Endocardial cells are marked by *Tg*(*kdrl*:NLS-mCherry) and *Tg(kdrl*:eGFP*)* expression, respectively showing their nuclei (yellow) and cytoplasm (cyan); individual cells were segmented using Imaris and assigned to the corresponding cardiac regions. (**B**), Individual segmented endocardial cells. (**C**), Average endocardial cell volume per heart in each defined region; mean ± SEM, one-way ANOVA followed by Tukey's post hoc test. (**D**), Number of AV endocardial cells in 48 hpf *egr3*^{*+*/*+*} and *egr3*^{*-*/*-*} embryos; Student's t test, mean ± SEM. (**C**, **D**), n = 5, 12, and 7 embryos; 1 experiment. AV = atrioventricular, V = ventricle, A = atrium. Scale bars (**A**) = 20 µm, (**B**) = 10 µm.

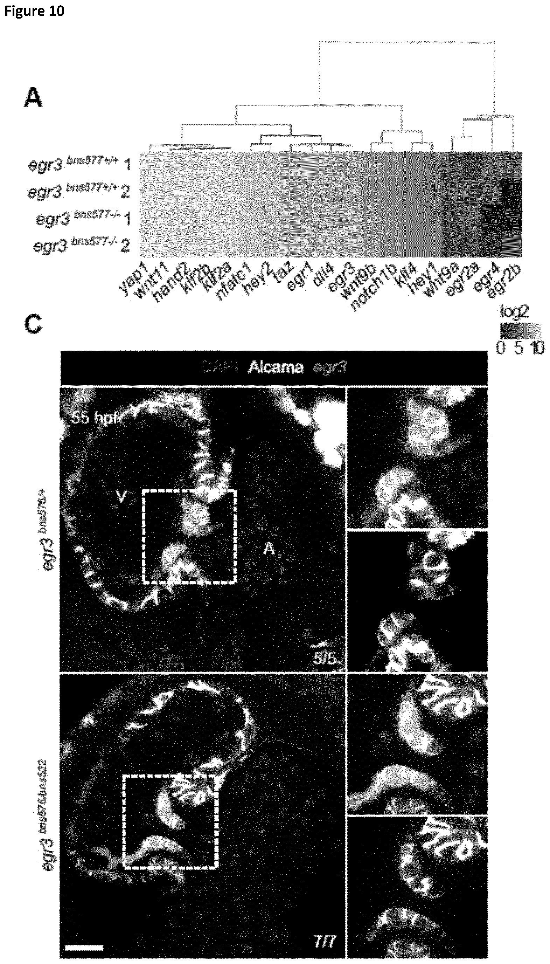

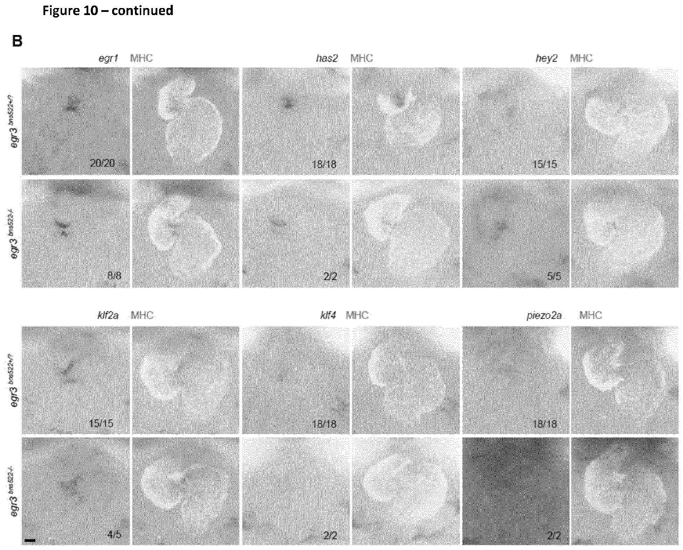

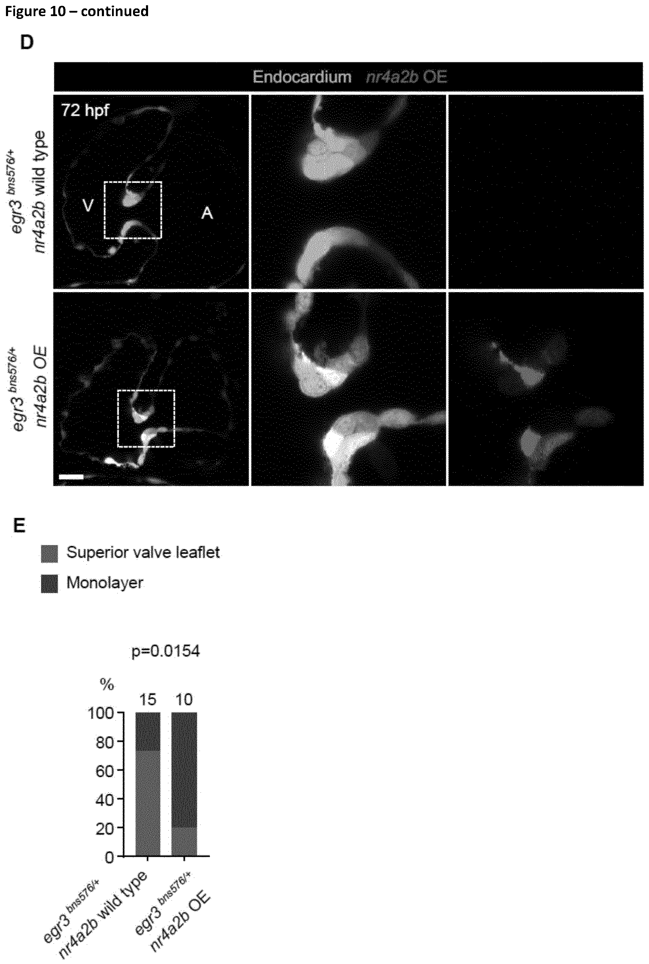

**Fig. 10****. *egr3* mutants display wild-type like patterning of the atrioventricular canal.** (**A**), RNA-seq heatmap analysis of key valve regulator genes. (**B**), *in situ* hybridization for different AV valve markers in 48 hpf *egr3*^{*+*/*+*} and *egr3*^{*-*/*-*} embryos. (**C**), Confocal images of representative Alcama immunostaining of hearts from 55 hpf *egr3*^{*bn1076*/*+*} and *egr3*^{*bn1076*/*bn1022*} sibling embryos. (**D**), Confocal images of representative hearts from control *Pt(egr3:Gal4-VP16)* and *egr3*-driven *nr4a2b* overexpressing *(Pt(egr3:Ga14-VP16); Tg(UAS:nr4a2b-p2a-dTomato)) egr3*^{*bn1076*/*+*} larvae at 72 hpf. **(E),** Percentage of control and egr3-driven *nr4a2b* overexpressing *egr3*^{*bn1076*/*+*} larvae with and without a superior AV valve leaflet (i.e., endocardial monolayer) at 72 hpf; n = 15 and 10; 2 independent experiments. Fisher's exact test. V = ventricle, A = atrium. Scale bars = 20 µm.

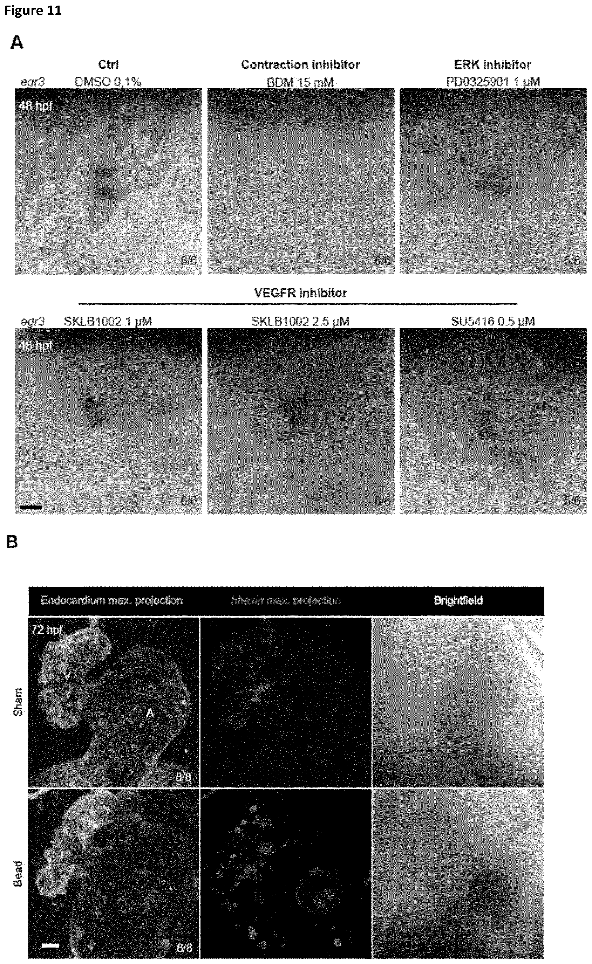

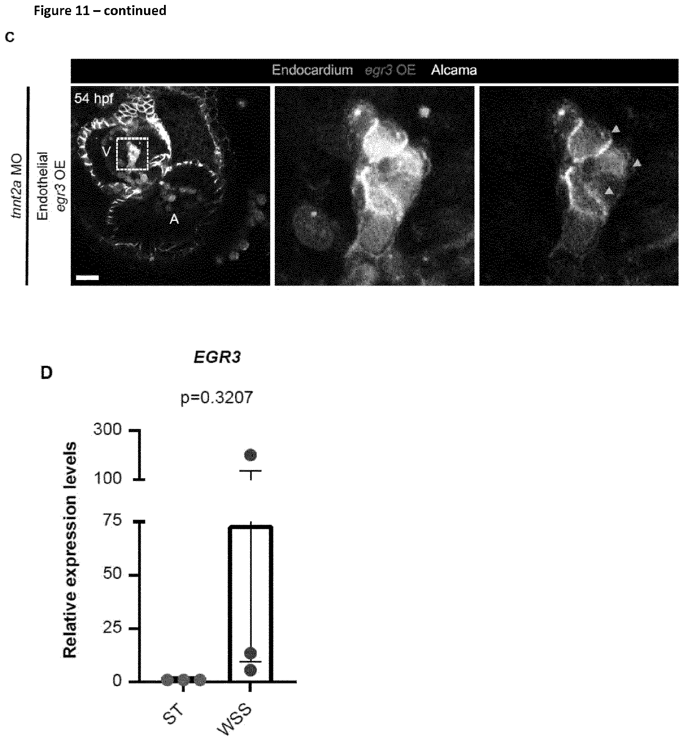

**Fig. 11****. Regulation of *egr3* cardiac valve expression.** (**A**), *in situ* hybridization for *egr3* expression following different chemical treatments. (**B**), Confocal images of representative hearts from sham control and bead-inserted larvae at 72 hpf; valve interstitial cells are marked by *Tg(Mmu.Hhex-*E1B:GFP*)* expression (magenta) and endocardial cells by Tg(kdrl:Hsa.HRAS-mCherry) expression (cyan). Red dotted circle highlights inserted bead. (**C**), Confocal images of representative hearts from an endothelial-specific *egr3* overexpressing *(Tg(flila:Gal4);Tg(UAS:Kaede);Tg(UAS:egr3-p2a-dTomato)) tnnt2a* MO injected embryo immunostained for Alcama expression at 54 hpf; magnified region shows Alcama expression in the ventricle of a *tnnt2a* MO injected *Tg(UAS:egr3-p2a-dTomato)* embryo. (**D**), Relative *EGR3* mRNA levels in porcine aortic valve endothelial cells (PAVECs) subjected to static (ST) or pulsatile wall shear stress (WSS) conditions; n = 3; 1 experiment. Student's t test, mean ± SEM. **(A, B and C)** Scale bars = 20 µm.

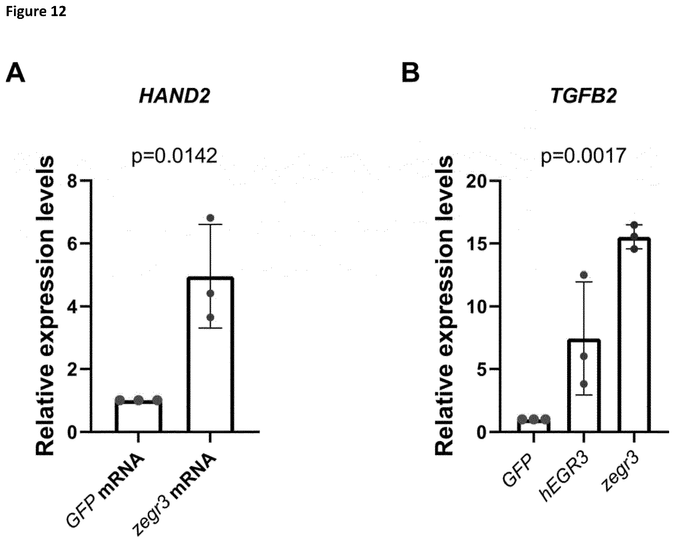

**Fig. 12****. *EGR3* overexpression is sufficient to induce valve differentiation in human endothelial cells. (A)** Relative mRNA levels of the valve marker gene *HAND2* in HUVECs transfected with *GFP* mRNA or zebrafish *egr3* mRNA. **(B)** Relative mRNA levels of the valve marker gene *TGFB2* in HUVECs transfected with a pSBbi plasmid containing a *GFP,* the CDS of the human *EGR3,* or the CDS of the zebrafish *egr3.* **(A, B)** Cells were analyzed 48 h post-transfection. Ct values are in Data S1. Student's t test, mean ± SEM. CDS = coding sequence.

The examples illustrate the invention.

### Example 1- Introduction

Tissue morphogenesis is intrinsically linked to the mechanical forces that are required for the cellular and molecular processes defining cell fate (*1-4*). In this context, endothelial cells, which line the blood vessels throughout the body, are specialized according to the function and mechanical properties of the tissues they reside in as well as blood flow patterns (*5-8*). During heart development, the specialized endothelial cells lining the cardiac wall, known as endocardial cells (EdCs), invade the adjacent extracellular matrix (ECM) in the atrioventricular (AV) canal and outflow tract (OFT) in response to oscillatory shear stress (*9-11*). These migrating EdCs acquire mesenchymal characteristics, differentiate into valve interstitial cells (VICs), and proliferate to give rise to the valve leaflets (*11-21*), which are essential for cardiac function as they guarantee unidirectional blood flow.

Extensive work in vertebrates has shown that perturbation of mechanical forces in the heart severely affects valve formation (*10, 22-25*). In particular, the zebrafish model allows for the observation and manipulation of intracardiac mechanical forces while imaging valve morphogenesis at single cell resolution. For example, stopping cardiac contraction, and consequently blood flow, results in a valveless phenotype (*26*). Molecularly, the expression of several genes has been shown to be flow sensitive (*11, 27*), and the classical endothelial flow-responsive transcription factor Klf2 has been described as the main mechanosensitive transcription factor required for cardiac valve formation (*9*, *10, 12, 16, 18, 28-31*). However, zebrafish *klf2a*/*b* double mutants only display a partially penetrant valveless phenotype (*12, 29, 32*), indicating that other transcription factors play critical roles in transducing the mechanical signals that promote valvulogenesis.

Herein, it is shown that the transcription factor Early growth response 3 (Egr3) is a critical transducer of mechanical signaling during cardiac valve morphogenesis in zebrafish. It is first shown that loss of *egr3* causes a complete and highly penetrant lack of cardiac valves. Data from loss- and gain-of-function experiments indicate that mechanical forces activate the expression of *egr3* in a subset of endocardial cells, thereby inducing their migration towards the adjacent ECM. Using transcriptomic analyses followed by functional investigation, it is also shown herein that the nuclear receptor Nr4a2b is a target and effector of Egr3 during cardiac valve formation. Furthermore, it is shown that mechanical forces upregulate *EGR3* expression in porcine valvular endothelial cells and that *EGR3* and its target/effector *NR4A2* may be involved in human cardiac valve remodeling upon ectopic biomechanical overload.

### Example 2 - Results

### Egr3 is a critical regulator of cardiac valve formation

Seeking to identify additional transcription factors required for cardiac valve formation in zebrafish, first the transcriptional landscape of endocardial cells was determined and explored at the time when key morphogenetic events underlying valve development take place. Thus, wild-type zebrafish hearts were isolated at 50 hours post-fertilization (hpf) when valve identity has just been established, and at 80 hpf when forming valves are first observed (*12-14, 33*) (Fig. 1A). Single-cell RNA-sequencing (scRNA-seq) of whole hearts was carried out and 1310 and 2194 endocardial cells at 50 and 80 hpf were obtained, respectively (Fig. 1B). The valve population was clearly identified at both time points by the expression of valve endocardium specific genes such as *ha7 (34)* and *alcoma (33)* (Fig. 6A). Differential expression analysis between valve and non-valve EdCs revealed *early growth response 3* (*egr3*) in the top 30 most enriched genes in the valve cluster and the only one encoding a DNA-binding protein with transcription factor activity (Fig. 1C). Notably, *egr3* expression appears more enriched in the valve endocardial cells when compared with other established valve transcription factor genes (*16, 18, 23, 35),* including *klf2a*/*b (29)* and *nfatc1 (14)* (Fig. 6, B and C). *Egr3* expression was further analyzed by *in situ* hybridization and observed a similarly restricted mRNA localization in the AV canal (Fig. 6D). Two other Egr family members have been implicated in cardiac valve development. In mouse, loss of *Egr2 (Krox20)* results in aortic valve defects and regurgitation (*36, 37*), while in zebrafish and humans, *egr1*/*EGR1* has been shown to be expressed in cardiac valves (*38, 39*) and this expression requires mechanical forces (*38, 40*). *Egr1* expression was observed mostly at 80 hpf and in most endocardial cells (Fig. 6, B and C) and neither *egr2a*/*b* nor *egr4* were detectable in our dataset (Fig. 6, B and C). Although the expression pattern and potential role of Egr3 in heart development have not been investigated yet, analyses of human and mouse heart scRNA-seq datasets (*41, 42*) reveal that *EGR3*/*Egr3* is expressed in cardiac valve endocardium and atrioventricular mesenchyme (Fig. 7, A and B). Sequence alignment shows that the Egr1-4 DNA binding domains are highly conserved (Fig. 7, C and D), indicating the potential existence of common targets.

Given the strong and early enrichment of *egr3* in the valve endocardium, its role in valvulogenesis was investigated by generating two mutant alleles using the CRISPR/Cas9 system, a 5.8 kb full locus deletion (*bn1022*) (i.e., a null allele), and an 11 base pair deletion (Δ11) (*bn1077*) resulting in a premature termination codon that is predicted to disrupt the DNA binding domain (Fig. 1D, Fig. 6, E and F). During early development, the valveless embryonic heart achieves unidirectional blood flow in part because of localized contraction waves and a simple vascular network (*43*) whose capacitance provides pressure storage and diminishes cardiac efforts (*44*). By 72 hpf, the AV endocardial cells have folded into a functional prevalvular structure capable of reducing retrograde blood flow and therefore supporting cardiac output (*10, 14, 15*). *egr3* mutants, of both alleles, are indistinguishable from their wild-type siblings until 72 hpf when they start to exhibit noticeable pericardial edema (Fig. 1, E and F). In contrast to their wild-type siblings, *egr3* mutants fail to form AV valve leaflets as their endocardial cells remain as a monolayer in a fully penetrant fashion in the null allele (19/19) and a nearly fully penetrant fashion in the Δ11 allele (19/21) (Fig. 1, G and H, Fig. 6, G and H). This complete lack of AV valves results in severe retrograde blood flow (Fig. 1I,) and leads to heart failure and impaired peripheral circulation (Fig. 1J,); *egr3* mutants indeed no longer display cardiac output by 96 hpf. Despite this impaired circulation, cardiac contraction and heart rate are not obviously affected in *egr3* mutants. In 96 hpf wild-type larvae, the AV valves are more mature and elongated and the forming outflow tract (OFT) valve leaflets are also present (*14*, *16, 45*). At this stage, the AV endocardial cells in *egr3* mutants still remain as a monolayer (Fig. 1, G-H, Fig. 6, G and H) and, similarly, the forming OFT valves also fail to develop (Fig. 1, K and L, Fig. 6I). Taken together, these data show that Egr3 is essential for cardiac valve formation in zebrafish.

### Endothelial cell-specific deletion of egr3 results in a lack of atrioventricular valves

To more precisely assess *egr3* expression in the developing heart, a Gal4 knock-in reporter line was generated (*Pt*(*egr3:Gal4-VP16*)), hereafter referred to as *egr3:Gal4* (*bn1076*), by targeting exon 2 using the CRISPR/Cas9 system, thereby disrupting the zinc-finger DNA binding domain (Fig. 2A). Consequently, the *egr3:Gal4* line has endogenous *egr3* reporter activity and is also an *egr3* mutant allele (Fig. 8B). *egr3* reporter⁺ embryos display a strong expression in the olfactory bulb as well as in specific structures of the central and peripheral nervous systems (Fig. 8A). *egr3* reporter expression is also evident in the presumptive AV canal starting at 34 hpf (Fig. 8A), mostly in the AV endocardium, but also in the AV myocardium (Fig. 2B). Moreover, *egr3* reporter expression is observed in the OFT endocardium starting at 72 hpf (Fig. 2B), coinciding with the onset of OFT valve morphogenesis (*16*). Notably, most *egr3* reporter expressing endocardial cells were observed in, or near, the AV canal or OFT.

Considering that both endocardial and myocardial cells in the AV canal express *egr3* based on the *egr3*:Gal4 reporter expression, it was sought to determine whether *egr3* deletion in one of these cell types was sufficient to recapitulate the *egr3* mutant phenotype. Thus, the CRISPR/Cas9 system was used to generate a sequential *loxP* site knock-in in the *egr3* locus, specifically 440 bp upstream of the TSS and 1.9 kb downstream of the stop codon (Fig. 2C). Injection of *Cre* mRNA into embryos from *egr3* ^{*flox*/+} zebrafish crossed with *egr3*^{*+*/*-*} zebrafish resulted in 72 hpf larvae displaying pericardial edema and no valve leaflets, correlating with the recombination of the *egr3* floxed allele (Fig. 8, D to F). No phenotype was observed in the uninjected larvae, indicating that the *egr3* floxed allele is functional. When crossing *egr3*^{*flox*/*+*} zebrafish with *egr3*^{*+*/*-*} zebrafish carrying an endothelial Cre driver *(kdrl:Cre),* it was observed that deleting *egr3* in endothelial cells was sufficient to recapitulate the global *egr3* mutant phenotype, thereby resulting in the complete lack of AV valve leaflets (Fig. 8G, Fig. 2, D and E). Moreover, when crossing *egr3*^{*flox*/*+*} zebrafish with *egr3*^{*+*/*-*} zebrafish carrying a myocardial Cre driver *(myl7:Cre),* it was observed that deleting *egr3* in myocardial cells led to no noticeable differences with their control siblings including in their valve leaflets (Fig. 8H, Fig. 2, D to F). Collectively, these data show that *egr3* functions in the endothelium to drive AV valve formation.

### egr3 directs AV canal endocardial cell migration through its target Nr4a2b

In order to gain more insight into the cellular and molecular causes leading to the absence of the AV valve in *egr3* mutants, *egr3*^{*+*/*?*} and *egr3*^{*-*/*-*} sibling embryos were analyzed at 48 hpf, a time when they cannot be visually distinguished from each other. At this stage, the AV canal is already morphologically and molecularly distinct from the atrial and ventricular chambers (33). Tissue convergence and a decrease in AV canal EdC size are important morphogenetic steps that precede AV valve formation (*46*, *47*). Thus, in order to evaluate possible morphological differences in the endocardium leading to the *egr3* mutant phenotype, EdC volume was analyzed in each cardiac region (Fig. 9, A and B). Consistent with published data (*47*), AV canal EdCs had a smaller volume when compared with ventricular and atrial EdCs (Fig. 9C). Notably, there were no differences in EdC cell number or volume in any cardiac region between *egr3* mutants and their egr3^{*+*/?} siblings (Fig. 9, C and D), such that *egr3* mutant EdCs were observed in expected numbers and underwent the cell shape changes typically observed during early AV valve morphogenesis.

To identify the molecular causes of the *egr3* mutant phenotype, bulk RNA sequencing analysis of 48 hpf *egr3*^{*+*/*+*} and *egr3*^{-/-} dissected hearts was performed (Fig. 3A). 154 genes were differentially expressed between *egr3* wild-type and mutant sibling embryos. Notably, important valve regulators such as *klf2a*/*b, notch1b, dll4, nfatc1,* or *wnt9a*/*b* did not appear to be differentially expressed in *egr3* mutants (Fig. 10A). Using *in situ* hybridization for *egr1, ho7, hey2, klf2a, klf4, piezo2a,* and immunostaining for Alcama, it was indeed observed that the patterning of the AV canal was obviously not affected in *egr3* mutants (Fig. 10, B and C), altogether suggesting that lack of *egr3* leads to the absence of the AV valve in spite of apparently unaffected expression of established cardiac valve markers and regulators. The dataset was further screened for differentially expressed genes with cardiac valve expression, and found that *nr4a2b, spp1,* and *nrg1* were significantly downregulated in *egr3* mutants (Fig. 3A). Nr4a2/Nurr1 is a nuclear receptor with transcription factor activity (48) and *nr4a2b* exhibits cardiac valve expression according to our scRNA-seq data (Fig. 3B), while *spp1*/*osteopontin* and *nrg1* are expressed by valve EdCs (*49, 50*) (Fig. 3, B and C). Our observations that *nr4a2b* and *spp1* are downregulated in *egr3* mutants agree with published reports in mammals showing that EGR3 is upstream of *Nr4a2* (*51*) and that NR4A2 activates *SPP1* expression by binding to its promoter (*52*). Moreover, EGR3 (*53, 54*), NR4A2 (*55*), and SPP1 (*56, 57*) promote endothelial cell migration downstream of VEGF, supporting the model that Egr3 is necessary for EdC migration during valvulogenesis.

To test whether deficient cellular migration could be the cause of the *egr3* mutant phenotype, EdC migration was evaluated in the initial steps of AV valve morphogenesis. From 48 hpf onwards, the AV canal EdCs start to extend protrusions into the adjacent ECM and, within a few hours, two to four AV canal EdCs collectively migrate in a ventricle to atrium direction (*13, 14*) (Fig. 3D). *egr3* mutants exhibit a two-fold reduction in the number of AV canal EdC protrusions towards the ECM (Fig. 3E), although this reduction was not significant. Strikingly, the subsequent collective EdC migration is severely affected, as only 6% (n=1/17) of the mutant embryos engaged in EdC migration compared with 72% (n=13/18) of their heterozygous siblings at 55 hpf (Fig. 3, F and G). Between two to four migrating EdCs were observed in these heterozygous hearts, while zero to two migrating EdC was generally observed in these *egr3* mutant hearts (Fig. 3H).

To investigate whether Egr3 is sufficient to induce EdC migration into the ECM, a stable *egr3* overexpression line was generated, *Tg(5XUAS:egr3-p2a-dTomato),* hereafter referred to as '*egr3* OE'. To induce ectopic *egr3* overexpression in the endothelium, *egr3* OE zebrafish was crossed with the endothelial *fli1a:Gal4* driver, *Tg(fli1a:Gal4FF). egr3* overexpressing cells were clearly distinguished by dTomato fluorescence (Fig. 4A). In control hearts, VICs were present in the ECM of the AV canal, but not in the ECM of the ventricle or atrium (Fig. 4, B and C). However, in *egr3* OE hearts, not only was there more than a 2.5 fold increase in VIC number in the ECM of the AV canal (Fig. 4, B and C), but dTomato⁺ EdCs were also present in the ventricular and atrial ECM, adjacent to the endocardial monolayer (Fig. 4, A to C). To better characterize the increased ECM invasion by EdCs in *egr3* OE hearts, the expression of the endocardial valve marker Alcama was assessed (*33*). Notably, *egr3* overexpression in the endothelium was sufficient to induce ectopic Alcama expression in ventricular and atrial endocardial cells (Fig. 4, D and E), as well as a significant increase of Alcama-expressing endocardial cells in the AV canal (Fig. 4E). Next, it was tested whether overexpressing the Egr3 target, Nr4a2b, would be sufficient to rescue AV canal EdC migration in *egr3* mutants. Thus, a stable UAS-driven *nr4a2b* overexpression line was generated, *Tg(5XUAS:nr4a2b-p2a-dTomato),* hereafter referred to as *'nr4a2b* OE', and used it in the background of the *egr3 bn1077* mutant allele and *egr3:Ga*/*4*^{*+*/}*⁻.* The resulting transheterozygous progeny fail to produce a functional Egr3 protein and overexpress *nr4a2b* in *egr3:*Gal4⁺ cells. Transheterozygous larvae displayed the expected *egr3* mutant phenotype at 72 hpf (Fig. 4F, Fig. 8B). *egr3* transheterozygous larvae overexpressing *nr4a2b* in their AV canal endocardium displayed a significant increase in the number of VICs in the AV canal ECM, indicating a partial rescue of EdC migration (Fig. 4, F and G). Not all VICs in *nr4a2b* OE hearts were dTomato⁺, indicating that Nr4a2b may also act in a cell non-autonomous fashion in this process. And as previously reported for *klf2a* overexpression in the AV canal, using a *nfatc:Gal4* line (*15*), it was found that *nr4a2b* has an inhibitory effect on valve formation in *egr3*^{*+*/*-*} larvae (Fig. 10, D and E), suggesting that *nr4a2b* expression needs to be tightly regulated during valvulogenesis. Collectively, these data indicate that the absence of cardiac valves in *egr3* mutants is a result of failed EdC migration into the adjacent ECM and that Egr3 is sufficient to induce endocardial Alcama expression and migration; they also suggest that Egr3 promotes AV canal EdC migration through its target Nr4a2b.

### egr3 is a mechanosensitive transcription factor gene in cardiac valve development and disease

Considering that EGR3 is involved in mediating VEGF-induced endothelial migration (*53*) and that it is a VEGF target via ERK activation (*58*), it was decided to test whether *egr3* AV canal expression was dependent on VEGF signaling. It was found that *egr3* AV canal expression appeared to be unaltered after treatment with VEGF receptor inhibitors or a MEK inhibitor (Fig. 11A), suggesting that it is independent of VEGF/ERK signaling. Given the fundamental role of mechanical signaling in cardiac valve formation (*10, 23, 26*), it was then decided to test whether *egr3* expression was downstream of mechanical forces. A no-flow/no-contraction condition as simulated *in vivo* by taking advantage of the zebrafish embryo's ability to withstand severe cardiac dysfunction (*59*). *Silent heart* zebrafish mutants were used which lack the sarcomeric protein Tnnt2a and, therefore, do not display cardiac contraction (*59*). Notably, *egr3* AV canal expression was completely abrogated in *tnnt2a* mutant hearts (Fig. 5A), and also the loss of *egr3* expression was observed when treating zebrafish embryos with the myosin inhibitor BDM (Fig. 11A). To test whether *egr3* expression responds to ectopic mechanical stimulation, microsurgical insertion of a bead inside the embryonic heart through the inflow tract (*22*, *60*) at 48 hpf was performed (Fig. 5B). The bead remained inside the heart for 24 hours and moved rhythmically across the ventricular chamber following the heartbeats and without stopping the blood flow. Upon this ectopic mechanical stimulation, *egr3* expression was expanded in the hearts of the bead-inserted larvae when compared with sham controls, as shown by the expression of the *egr3:Gal4* reporter (Fig. 5, C and D). No ectopic EdCs in the ECM or an increased number of VICs in the bead inserted hearts when compared with sham controls were found (Fig. 11B). As mechanical forces are required for valve formation, it was next tested whether overexpressing *egr3* in endothelial cells in no-flow conditions could rescue some of the early steps of valve development. *Tnnt2a* morpholinos (MO) were injected into one-cell stage embryos of *egr3* OE zebrafish crossed with the endothelial *flila:Gal4* driver (Fig. 5E). Valvular cell identity is absent in no-flow condition hearts as the endocardium remains as a monolayer and the expression of valve markers is absent (*26, 61*) (Fig. 5E). Notably, some *egr3-*overexpressing endothelial cells in no-flow condition hearts expressed Alcama, displayed a partly cuboidal valvular cell shape, and were often found in the ECM (Fig. 5E). These Alcama+ *egr3-*overexpressing cells were present in the endocardium of both the ventricle and atrium as well as in the AV canal of no-flow condition hearts (Fig. 5F, Fig. 11C), suggesting a lack of spatial specification of valve identity in no-flow hearts and/or the ability of Egr3 to drive this specification on its own.

To investigate whether *EGR3* is activated in mammalian valvular endothelial cells (VECs) in response to shear stress, a unique fluid activation device was used specifically designed to expose cells to physiologically relevant pulsatile wall shear stress (WSS) conditions (*62*). An upregulation of *EGR3* expression was observed in porcine VECs exposed to pulsatile WSS compared with static conditions (Fig. 11D), indicating that *EGR3* responds to pulsatile shear stress in VECs. In addition to playing an important role in valvulogenesis, mechanical forces are also pivotal during pathological valve remodeling. For instance, the use of a left ventricular assist device (LVAD) has become an established treatment option as well as a bridge for end-stage heart failure patients awaiting a heart transplant (*63*). Despite improving survival and heart function, LVAD use is correlated with aortic insufficiency, and 15 to 52% of patients may present worsening of previous aortic valve pathology or develop de *novo* aortic valve insufficiency (*64*). The LVAD increases cardiac output by diverting the blood from the left ventricle straight into the aorta, thereby decreasing the strain in the mitral valve and causing biomechanical overload in the aortic root and valves (*65*). This increased biomechanical overload in the aortic valves induces valve remodeling (i.e., aortic valve fusion) (*66, 67*). However, how the aortic valves transduce the biomechanical overload into a molecular response remains elusive. As zebrafish *egr3* expression responds to lack of, as well as increase in, mechanical forces during valve development (Fig. 5, A to D) and *EGR3* responds to pulsatile WSS in VECs (Fig. 11D), it was sought to investigate whether *EGR3* expression was altered during human aortic valve remodeling following LVAD placement. Samples of aortic valves were collected from control donors and LVAD patients and performed RT-qPCR (Fig. 5H). *EGR3* expression was increased by almost six-fold in LVAD patient valves compared with donor control valves (Fig. 5I). Expression of the classical mechanosensitive transcription factor gene *KLF2* was increased by nearly four-fold (Fig. 5I). Also EGR3 targets were tested and it was observed that in LVAD patient valves, *NR4A2* expression was increased by more than 13-fold and *SPP1* expression by more than 20-fold, although the latter was not significant (Fig. 5I).

### EGR3 overexpression is sufficient to induce valve differentiation in human endothelial cells

To access whether *EGR3* expression is sufficient to induce valve differentiation from endothelial human cells, human umbilical vein endothelial cells (HUVECs) were first transfected with zebrafish *egr3* mRNA or *GFP* mRNA as control. Zebrafish *egr3* mRNA transfection induced nearly a five-fold increase in gene expression of the valve marker *HAND2* (L. Cheng, M. Xie, W. Qiao, Y. Song, Y. Zhang, Y. Geng, W. Xu, L. Wang, Z. Wang, K. Huang, N. Dong, Y. Sun, Generation and characterization of cardiac valve endothelial-like cells from human pluripotent stem cells. Commun Biol 4, 1039 (2021) 48 h post-transfection (Fig. 12 A). Next, it was assessed whether human *EGR3* would also display the same potential in inducing valve differentiation. HUVECs were transfected with a pSBbi-GFP plasmid containing human *EGR3* or zebrafish *egr3* coding sequences, and empty pSBbi-GFP plasmid as control. Both human *EGR3* and zebrafish *egr3* transfections induced a significant increase in the expression of the valve marker *TGFB2* (Cheng et al., loc. lit.) 48 h post-transfection (Fig. 12 B). These data suggest that *EGR3* induces valve differentiation in human endothelial cells.

### Example 3 - Discussion

Herein the identification of the transcription factor Egr3 as a master transducer of the mechanical forces that guide valvulogenesis in zebrafish it described. It is shown that Egr3 is required for EdC migration into the adjacent ECM by activating the expression of the migration regulator *nr4a2b* (*55*). *Klf2* has long been considered to be the main mechanosensitive transcription factor gene in cardiac valve formation. However, the broad endocardial expression of *klf2a*/*b* compared with the restricted expression of *egr3* in valve EdCs suggests that Egr3 has a more specific role in transducing mechanical forces during valvulogenesis. In fact, *klf2* mutations in zebrafish result in a 47-56% penetrance of the cardiac valveless phenotype observed in no-flow conditions (*29, 32*). Moreover, *klf2* mutants also display cardiomyocyte extrusion (*32*) and it is not clear how this phenotype affects cardiac valve formation. In contrast, the *egr3* mutant phenotype is highly penetrant and it is specific to the valves, with no obvious defects in other cardiac regions.

*EGR3* is expressed in AV canal EdCs and fibroblasts in mammalian hearts (*41, 68*) and it responds to pulsatile WSS in VECs, consistent with a conserved function in valvulogenesis. *Egr3* mutant mice display an increased frequency of perinatal mortality (*69*), and it would be worth investigating them for any underlying cardiac defects. In humans, *EGR3* single nucleotide variants resulting in loss-of-function alleles are highly underrepresented with a loss-of-function observed/expected upper bound fraction of 0.396 (gnomAD v4.0.0), possibly suggesting that *EGR3* is haploinsufficient. Haploinsufficiency is also typically observed in genes associated with bicuspic aortic valve disease, such as *GATA6* (*70*) and *NOTCH* (*71, 72*). Of note, case reports have shown that chromosomal duplications of the region where *EGR3* is located (8p21.3) are associated with congenital heart disease (i.e., valve and septal defects) (*73, 74*), while a case-control study has reported an association between an *EGR3* locus polymorphism and coronary artery disease (CAD) (*75*), though with no mention of possible valve defects.

In parallel to *egr3* upregulation following mechanical stimulation in the developing zebrafish heart, *EGR3* is upregulated in porcine VECs and in LVAD aortic valves upon biomechanical overload. In addition, *EGR3* expression does not seem to respond to fluid shear stress in HUVECs (*76*), suggesting a cell- and/or flow-specific response. In addition, EGR3 is necessary and sufficient to partially mediate the TGF-β fibrotic response in fibroblasts (*77*). Thus, it can be assumed that the biomechanical stimuli that promote *EGR3* expression during cardiac development also promote it in LVAD aortic valves, thereby contributing to pathological aortic valve remodeling and insufficiency (*65-67*). It will be important to investigate the role of *EGR3, KLF2,* and other mechanosensitive transcription factor genes in LVAD aortic valve pathogenesis.

Transduction of mechanical forces, which is mediated in part by VEGF signaling (*78*), also plays a role in lymphatic endothelial cell (LEC) development and physiology. VEGF is a known regulator of *EGR3* and *NR4A2* expression in endothelial cells (*53, 58*, *79*). In fact, human LECs upregulate *EGR3* and *NR4A2* expression following VEGF stimulation (*80, 81*), and a recent report on the transcriptional coactivator Zmiz1 suggests that *Egr3* is also involved in lymphatic valve formation (*82*).

In summary, our study reveals a previously unknown signaling axis in which EGR3 is necessary to transduce the mechanical signals required for cardiac valve formation and potentially also for LVAD valve remodeling. It is expected that perturbations in the function or expression of *EGR3,* or its targets, also lead to cardiac valve defects in humans.

### Example 4- Materials and Methods

### Zebrafish handling and lines

All zebrafish husbandry was performed under standard conditions in accordance with institutional (Max Planck Gesellschaft) and national (German) ethical and animal welfare regulations. All procedures performed on animals conform to the guidelines from Directive 2010/63/EU of the European Parliament on the protection of animals used for scientific purposes and were approved by the Animal Protection Committee (Tierschutzkommission) of the Regierungspräsidium Darmstadt (reference: B2/1218). The following mutant, knock-in reporter, and transgenic lines were generated for this study: *egr3^{bn1022}, egr3^{bn1077}, Pt(egr3:Gol4-VP16)^{bn1076}* abbreviated as *egr3:Gal4, Pt(egr3:loxP-egr3-loxP)^{bn1161}, Tg(5xUAS:egr3-p2a-dTomato)^{bn1107}* abbreviated as *egr3:OE, Tg(5xUAS:nr4a2b-p2a-dTomato)^{bns719}* abbreviated as *nr4a2b:OE.* The following transgenic and mutant lines were used in this study: *tnnt2a^{mn0031Gt} or Gt(GBT-R14)⁽⁸³⁾, Tg(kdrl:EGFP)*^{*S8a3* (*33*)}, *Tg(kdrl:Hsa.HRAS-mCherry)*^{*s896* (*84*)}*, Tg(kdrl:NLS-mCherry)*^{*i9* (*85*)}*, Tg(fli1a:Gal4FF)^{ub9 (86)}, Tg(myl7.-BFP-CAAX)*^{*bn693* (*87*)}*, Tg(myl7:mCherry-CAAX)^{bns7 (88)}, Tg(myl7:EGFP)*^{*twu26* (*89*)}*, Tg(5xUAS:EGFP)^{nkuasgfp1a (90)}, Tg(UAS:Kaede)^{rk8 (91)}*, *Tg(myl7:Cre)^{sd55 (92)}, Tg(kdrl:Cre)*^{*s898* (*93*)}*, Tg(Mmu.Hhex-E1B:GFP)^{bn821(14)}.*

### Generation of zebrafish lines

For Crispr/Cas9 mutagenesis, *in vitro* synthesis of Cas9 mRNA and design of gRNAs were performed as previously described (*94, 95*). gRNA efficiency was evaluated in single AB wild-type injected embryos by High Resolution Melting Analysis (HRMA) or T7 endonuclease assay. Generation of *egr3* full locus deletion (*bn1022*) was achieved by using one gRNA in the immediate 5' intergenic region and another in the 3' UTR, and F1 adults were screened by PCR followed by sequencing of PCR products. The *egr3* Δ11 allele (*bn1077*) was generated with commercially available *egr3* gRNA (IDT), and F1 adults were screened by HRMA followed by sequencing of PCR products. The *egr3:Gal4* reporter line (*bn1076*) was generated according to the GeneWeld method (*96*) with 48 bp homology arms flanking the *p2a-Gal4-VP16-beta-actin* sequence, and using the *egr3* gRNA (IDT) as for the *egr3* Δ11 allele (*bn1077*). The previously published sequential *loxP* knock-in method (*97*) was used to generate the *egr3* floxed allele (*bn1161*) with single-stranded donor oligonucleotides containing *loxp* sites flanked by asymmetric 21 and 49 bp homology arms. For the 5' *loxP* knock-in the same 5' gRNA as for the full locus deletion allele (*bn1022*) was used. Adult F1 zebrafish were screened for the 5' *loxP* integration using a *loxP-*specific PCR primer and sequenced after performing a *loxP*-flanking PCR. The 5' *loxP* founder zebrafish was then crossed with AB wild type and the resulting one-cell stage embryos were injected with a 3' gRNA designed to cut the neighboring intergenic region. Adult F1 *egr3* floxed allele zebrafish were screened by *loxP*-specific and flanking PCRs followed by sequencing of PCR products. Despite observing a complete and adequate integration of the 5' *loxP* site, it was not possible to sequence the full extent of the 3' *loxP* site. Genetic polymorphisms and/or integration of multiple copies after mutagenesis of the 3' region in the zebrafish used for 3' *loxP* knock-in might explain why it was not possible to sequence the *loxP* integration. However, the floxed *egr3* allele was confirmed by the *loxP-*specific and flanking PCRs, as well as by functional analysis after *Cre* mRNA injection, in which a recombined band of 400 bp was identified by PCR and the *egr3* phenotype was observed at the expected Mendelian ratios. Conditional knockouts were obtained by crossing *egr3*^{*flox*/*+*} zebrafish with *egr3*^{*bn1077*/*+*} zebrafish carrying *Tg(kdrl:Cre)* or *Tg(myl7:Cre),* for endothelial or myocardial cell-specific knockout, respectively. The low number of myocardial cell-specific knockout larvae (3/46) (Fig. 2D and F) is under the expected ratio of 1/8 for a cross of *egr3*^{*bn1077*/}*⁺, egr3*^{*flox*/*+*} and *myl7:Cre*^{*+*/*-*} zebrafish and without selecting the progeny for a phenotype. *Tol2* transgenesis was used to generate the *egr3 (bn1107)* and *nr4a2b* (*bns719)* overexpression lines, respectively *egr3:OE and nr4a2b:OE.* Adult *egr3:OE and nr4a2b:OE* F1 zebrafish were screened by crossing with *Tg(fli1a:Gal4FF)* zebrafish and using the dTomato⁺ expression in the progeny as a proxy. dTomato⁺ embryos were used to confirm transgenesis by PCR amplification and sequencing of the PCR products. Of note, the data presented in this work were generated using progeny from F1 to F3 animals (obtained from sequential outcrosses to different transgenic lines or non-transgenic AB zebrafish); while doing the revisions, a reduction in the penetrance of the cardiac valve phenotype in the progeny of F4 animals was observed. Genotyping PCRs were performed with KAPA2G Fast Ready Mix (Sigma 2GFRMKB) and HRMAs with Maxima SYBR Green/Fluorescein qPCR Master Mix (2X) (Thermo Scientific K0241).

### Plasmid construction

The ORFs for *egr3, nr4a2b, spp1, nrg1, hey2,* and *ha7* were amplified from cDNA generated from a pool of 78 hpf larvae, and cloned into a pC7+ vector. To generate the *egr3:Gal4* line, the *pGTag-Gal4-VP16-beta-actin* plasmid was a gift from Jeffrey Essner (Addgene # 117817) and homology arms were added by vector ligation (98). To generate the *egr3:OE* line *(bn1107)* and *nr4a2b:OE* line *(bns719), egr3* and *nr4a2b* were amplified from pC7+ and cloned into a *5XUAS:p2a-dTomato* vector. Vector ligation was performed by PCR amplification followed by *in vivo* cloning(99).

### Microscopy

Live imaging of stopped zebrafish hearts was performed by mounting PTU-treated embryos and larvae in 1% agarose containing 0.2% tricaine. The following confocal microscopes were used: LSM700 Axio Imager 2 and LSM880 Axio Examiner with a W Plan-Apochromat 40×/1.0 dipping lens for image acquisition. Fiji (ImageJ 2.1.0/1.53o) was used to adjust the brightness and contrast of representative images. For three-dimensional rendering and analysis of confocal images, Imaris x64 (Bitplane, 10.0.1) was used. The Imaris cell identification function was used to automatically segment endocardial cells based on their nucleus, marked by *Tg*(*kdrl*:NLS-mCherry) expression, and their cytoplasm, marked by *Tg(kdrl:EGFP)* expression. The endocardial cells where further segregated into ventricular, atrioventricular and atrial with the help of the x/y position object filtering followed by manual curation. Illustrative cartoons were created using Inkscape vector graphics editor unless otherwise indicated in the Figure Legends.

### In situ hybridization and immunostaining

Whole-mount RNA *in situ* hybridization was performed as previously described (*100*). *in situ* probes for *egr3, spp1, nrg1, hey2,* and *ha7* were synthesized from previously cloned ORFs in pC7+ vectors using PCR products with an incorporated T7 promoter. An *in situ* probe for *egr1* was kindly provided by Julien Vermot in a pBSK vector. *in situ* probes for *klf2a (15), klf4, nr4a2b, piezo2a* (*15*) were synthesized directly from a PCR product of 78 hpf wild-type AB cDNA, using primers with an incorporated T7 promoter. To visualize the cardiac outline, the primary Mouse anti-MHC antibody (1:500, MF-20, DSHB AB_2147781) was used, followed by the secondary Goat anti-Mouse IgG Alexa Fluor 488 antibody (1:500, Invitrogen A11029). To assess atrioventricular valve identity, immunostaining for Alcama using Mouse anti-Alcama antibody (1:50, ZN-8, DSHB AB_531904) was performed followed by Goat anti-Mouse-Alexa 647 antibody (1:500, Invitrogen A21236). *In situ* hybridizations were imaged using an SMZ25 stereomicroscope (Nikon) with a 2x/0.3 objective.

### AV blood flow fraction

Functional analysis of blood regurgitation was performed in 72 hpf zebrafish larvae mounted in 1% agarose without tricaine. The zebrafish hearts were imaged live using an inverted Cell Observer Spinning Disk microscope with a 25x/0.8 water-immersion objective at 240 frames per second (fps). Quantification of AV blood flow fraction was performed using a custom-made ImageJ script as previously described (*15*). For each imaged larva, AV retrograde blood flow is shown as a fraction of three averaged cardiac cycles.

### Microsurgical insertion of a bead

To assess the influence of ectopic mechanical stimulation on *egr3* expression *in vivo,* a bead was kept inside of the beating zebrafish heart for 24h (*22, 60*). For that manipulation, 48 hpf zebrafish embryos were mounted ventrally in 1% agarose and a microsurgical incision was performed in the yolk-sac using thin forceps. A bead (Cube Biotech 32201) was inserted with the forceps into the yolk cavity and gently pushed into the inflow tract, eventually reaching the heart through suction. Embryos were released from the agarose and kept in egg water at 28.5°C for 24 h, when *egr3*:Gal4; *UAS*:eGFP expression was assessed by confocal microscopy. Only larvae that had the bead inside of the ventricle and still displayed blood flow were considered for the analysis. Sham embryos underwent the same procedures of mounting and yolk piercing, but without bead insertion.

### Chemical treatments

To evaluate the influence of cardiac contraction on *egr3* expression, wild-type embryos were treated with 15 mM of the myosin inhibitor BDM (Sigma B0753). Possible regulation of AV *egr3* expression by VEGF signaling was tested by treating embryos with 1 µM and 2.5 µM of SKLB1002 (Selleck Chemicals S7258) (*101*) and 0.5 µM of SU5416 (Sigma S8442). Possible regulation of AV *egr3* expression by ERK signaling was tested by treating embryos with 1 µM of the MERK inhibitor PD0325901 (Sigma PZ0162) (*2*). Concentrations were adjusted in order to diminish side effects. 0.1% DMSO was administered as vehicle and all treatments were performed from 36 to 48 hpf, when embryos were collected and fixed for *in situ* hybridization.

### Porcine aortic valve endothelial cell isolation and culture

A fluid activation device that applies physiologically relevant pulsatile wall shear stress (WSS) was used on the surface of porcine aortic valvular endothelial cells (VECs) as previously described (*62*). VECs were isolated from porcine aortic valve leaflets. Briefly, aortic valve leaflets were isolated and incubated in collagenase type II/DMEM (Life Technologies) for 3x7 minutes. After incubation, cells were gently scraped to isolate VECs from the two sides of the leaflets. After isolation, VECs were seeded and cultured on flask coated with 50 µl/ml rat collagen type I (10 mg/ml concentration, BD Biosciences). VECs were cultured at 37°C and 5% CO2 in Endothelial Cell Growth Medium (Promocell) supplemented with manufacturer's adjuvants and 5% FBS. After 1 h of culture, VECs were first removed from the collagen gels and lysed in Trizol (Thermo Scientific 15596026). RNAs were extracted using RNeasy miniKit (Qiagen 74104).

### Research ethics for donated aortic valve samples

The study population consisted of left ventricular assist device (LVAD; n=6) patients referred for heart transplantation at La Timone Hospital (*102*). Patients were not included if they had connective tissue disease and previous aortic valve dysfunction. The patients provided their written informed consent to participate in this study (approved by the Marseille ethics committee n°13.061). Aortic regurgitation (AR) was graded through an integrative approach (*103, 104*). Mechanism of AR was separated into prolapse of the fused leaflet, cusp restriction or both. Aortic valves were recognized by their anatomical landmarks under the microscope, and leaflets were isolated with minimal aortic wall contamination. Immediately following surgical removal, a portion of the aortic valve leaflet was placed in RNAlater solution (Sigma) and stored at -80°C until processing. Control valve samples were obtained at autopsy of individuals without cardiac problems who suffered a traumatic death (n=8). The control group was composed of individuals with an average age of 59 +/- 4 years, and a male to female ratio (M/F) of 85%. The LVAD group was composed of individuals with an average age of 54 +/- 12 years, and M/F ratio of 71%. This study was performed according to the principles of the Declaration of Helsinki and in accordance with institutional guidelines.

### qRT-PCR

Total RNA from human aortic valve samples was purified using Trizol (Life Technologies) and RNeasy mini Kit. RNA was reverse transcribed using the AffinityScript Multiple Temperature cDNA synthesis kit (Agilent). Quantitative PCR was performed using SYBR Green (Roche) based qPCR on a LightCycler 480 (Roche). Graphs show the mean +/- standard deviation for 6-8 biological replicates. Samples were normalized to *TBP* or *GAPDH* as endogenous housekeeping genes. Relative expression levels were calculated by the comparative cycle threshold (ΔΔCt) method.

### scRNA-seq sample preparation and data analysis

In order to obtain a longitudinal transcriptional signature of single endocardial cells, hearts from *Tg(myl7:EGFP)* wild-type zebrafish were isolated at 50 and 80 hpf. Up to 150 hearts were isolated per condition as previously described (*105*). Briefly, zebrafish embryos and larvae were fragmented using a needle and syringe in an Eppendorf tube with 1 ml of DMEM (Thermo Scientific 88281) with 10% FBS (Sigma F2442). The homogenate was filtered through a 100 µm mesh filter. An additional wash with DMEM with 10% FBS was performed to remove the remaining hearts in the Eppendorf tube, followed by filtering. The isolated hearts were manually collected from the flow-through under a fluorescence microscope. The hearts were further dissociated using the Pierce Primary Cardiomyocyte Isolation Kit (Thermo Scientific 88281) and incubated for 25 minutes at 30°C (shaker 300 rpm) with pipetting every 5-10 minutes. Dead cells were removed from the final cell isolate by FACs sorting in PBS without Calcium and Magnesium (Lonza, 17-516F) and with 0.04% BSA. The cell suspensions were counted with a Moxi cell counter and diluted according to manufacturer's protocol to obtain 10.000 single cell data points per sample. Each sample was run separately on a lane in a Chromium controller with Chromium Next GEM Single Cell 3' Reagent Kits v3,1 (10×Genomics). scRNA-seq library preparation was done using a standard protocol, and sequencing was done on a Nextseq2000. scRNA-seq analysis was performed as previously described (*106*) and final data visualization was done using a CellxGene package (doi:10.5281/zenodo.3235020). Endocardial cells were identified by the expression of endothelial markers (e.g., *cdh5, fli1a, kdrl, tie1*) and represented 3504 from the 6476 sequenced heart cells. Valve endocardial cells were identified by the expression of valve markers (e.g., *alcama, ho7)* and represented 1343 cells. Data analysis was performed using only the endocardial cells, and the other cell types are not displayed for visualization purposes. The classification for transcription factor used was based on the GO terms containing direct DNA-binding domain, such as "DNA-binding transcription factor activity, RNA polymerase II-specific" and "DNA binding". Thus, *crip2* did not appear in our gene enrichment analysis as the encoded protein seems to lack a direct DNA binding domain (*107*).

### RNA-seq sample preparation and data analysis

To further investigate the *egr3* mutant phenotype, a bulk RNA-seq analysis of dissected zebrafish hearts was conducted at 48 hpf. As *egr3* mutants cannot be distinguished from their siblings at this stage, embryos were live genotyped at 36 hpf as previously described (*108*). 20 hearts were dissected for each biological duplicate of *egr3*^{*bn1077-*/*-*} and *egr3*^{*bn1011+*/*+*} sibling samples in cold DMEM with 10% FBS. Total RNA was isolated using the miRNeasy micro Kit (Qiagen 217084), followed by on-column DNase digestion (DNase-Free DNase Set, Qiagen 79254). Final elution was performed in 12 µl of RNase-free water, and subsequent RNA quality control, cDNA preparation, sequencing, and analyses were performed as previously described (*101*). A total of 154 genes were significantly dysregulated based on a padj-value<0.05. Heatmaps were obtained using the Webbased Interactive Omics visualizatioN - Applications (WlIsON) (*109*)*.*

### Statistical analysis

GraphPad Prism (v.9) was used to perform all statistical analyses. Two-tailed Student's t-test was used for comparing two samples (Fig. 3, H, Fig. 4, C, E and G, Fig. 5, D, F and I, Fig. 9D, Fig. 11D), and one-way ANOVA followed by Tukey's post hoc test for multiple comparisons (Fig. 1I, Fig. 9C); data are presented as mean ± SEM and actual p-values are shown. Two-sided fisher's exact test was used to compare categorical data between two groups (Fig. 1H and J and L, Fig. 2, E and F, Fig. 3E and G, Fig. 8C, Fig. 10E) and data were represented as percentages after statistical analysis to improve visualization. To allow for statistical analysis, the classifications "Superior valve leaflet" and "Superior s-shape leaflet" were collapsed into "Superior valve leaflet", and the "Monolayer / one VIC" and "Monolayer" were collapsed into "Monolayer" (Fig. 1H); all classifications and proportions are in Fig. 6, G to I and Fig. 8I. All genetic experiments with zebrafish were performed independently at least twice, using different batches of embryos and on different days unless otherwise indicated. Exact sample sizes and p-values are described in the figures and figure legends. p-values<0.05 were considered significant.

### HUVEC cell culture and transfection

Human umbilical vein endothelial cells (HUVECs) were purchased from (Lonza C2519A) and used until the sixth passage. HUVECs were cultured in Endothelial Cell Growth Medium (Sigma-Aldrich 211-500) with 100 units/ml of penicillin and 100 µg/ml of streptomycin. Cell transfection of *GFP* mRNA and zebrafish *egr3* mRNA were performed using the Lipofectamine^{™} MessengerMAX^{™} Reagent (ThermoFisher LMRNA001) according to the manufacturer's protocol. Cell transfection of pSBbi-GFP plasmids (Addgene 60511) empty (control), containing human *EGR3* or zebrafish *egr3* were performed using the Lipofectamine^{™} 3000 Reagent (ThermoFisher L3000001) according to the manufacturer's protocol. The day before transfection, cells were detached, counted and 4 × 10⁴ cells were seeded per well on a 24-well plate in 0.5 ml of complete growth medium, to achieve 50~80% of confluence on the day of transfection.

### References

1. H. Vignes, C. Vagena-Pantoula, J. Vermot, Mechanical control of tissue shape: Cell-extrinsic and -intrinsic mechanisms join forces to regulate morphogenesis. Semin Cell Dev Biol 130, 45-55 (2022).
2. R. Priya, S. Allanki, A. Gentile, S. Mansingh, V. Uribe, H. M. Maischein, D. Y. R. Stainier, Tension heterogeneity directs form and fate to pattern the myocardial wall. Nature 588, 130-134 (2020).
3. M. Daems, H. M. Peacock, E. A. V. Jones, Fluid flow as a driver of embryonic morphogenesis. Development 147, (2020).
4. P. Sidhwani, D. Yelon, Fluid forces shape the embryonic heart: Insights from zebrafish. Curr Top Dev Biol 132, 395-416 (2019).
5. E. Trimm, K. Red-Horse, Vascular endothelial cell development and diversity. Not Rev Cardiol 20, 197-210 (2023).
6. C. Aitken, V. Mehta, M. A. Schwartz, E. Tzima, Mechanisms of endothelial flow sensing. Nature Cardiovascular Research 2, 517-529 (2023).
7. H. Nakajima, A. Chiba, M. Fukumoto, N. Morooka, N. Mochizuki, Zebrafish Vascular Development: General and Tissue-Specific Regulation. J Lipid Atheroscler 10, 145-159 (2021).
8. R. N. Das, Y. Tevet, S. Safriel, Y. Han, N. Moshe, G. Lambiase, I. Bassi, J. Nicenboim, M. Bruckner, D. Hirsch, R. Eilam-Altstadter, W. Herzog, R. Avraham, K. D. Poss, K. Yaniv, Generation of specialized blood vessels via lymphatic transdifferentiation. Nature 606, 570-575 (2022).
9. E. Heckel, F. Boselli, S. Roth, A. Krudewig, H. G. Belting, G. Charvin, J. Vermot, Oscillatory Flow Modulates Mechanosensitive klf2a Expression through trpv4 and trpp2 during Heart Valve Development. Curr Biol 25, 1354-1361 (2015).
10. J. Vermot, A. S. Forouhar, M. Liebling, D. Wu, D. Plummer, M. Gharib, S. E. Fraser, Reversing blood flows act through klf2a to ensure normal valvulogenesis in the developing heart. PLoS Biol 7, e1000246 (2009).
11. A. O'Donnell, K. E. Yutzey, Mechanisms of heart valve development and disease. Development 147, (2020).
12. E. Steed, N. Faggianelli, S. Roth, C. Ramspacher, J. P. Concordet, J. Vermot, klf2a couples mechanotransduction and zebrafish valve morphogenesis through fibronectin synthesis. Not Commun 7, 11646 (2016).
13. F. Gunawan, A. Gentile, R. Fukuda, A. T. Tsedeke, V. Jimenez-Amilburu, R. Ramadass, A. lida, A. Sehara-Fujisawa, D. Y. R. Stainier, Focal adhesions are essential to drive zebrafish heart valve morphogenesis. J Cell Biol 218, 1039-1054 (2019).
14. F. Gunawan, A. Gentile, S. Gauvrit, D. Y. R. Stainier, A. Bensimon-Brito, Nfatc1 Promotes Interstitial Cell Formation During Cardiac Valve Development in Zebrafish. Circ Res 126, 968-984 (2020).
15. T. Juan, A. Ribeiro da Silva, B. Cardoso, S. Lim, V. Charteau, D. Y. R. Stainier, Multiple pkd and piezo gene family members are required for atrioventricular valve formation. Not Commun 14, 214 (2023).
16. A. L. Duchemin, H. Vignes, J. Vermot, Mechanically activated piezo channels modulate outflow tract valve development through the Yap1 and Klf2-Notch signaling axis. Elife 8, (2019).
17. J. Bischoff, Endothelial-to-Mesenchymal Transition. Circ Res 124, 1163-1165 (2019).
18. A. Paolini, F. Fontana, V. C. Pham, C. J. Rodel, S. Abdelilah-Seyfried, Mechanosensitive Notch-DII4 and Klf2-Wnt9 signaling pathways intersect in guiding valvulogenesis in zebrafish. Cell Rep 37, 109782 (2021).
19. J. Lincoln, V. Garg, Etiology of valvular heart disease-genetic and developmental origins. Circ J 78, 1801-1807 (2014).
20. J. Pestel, R. Ramadass, S. Gauvrit, C. Helker, W. Herzog, D. Y. Stainier, Real-time 3D visualization of cellular rearrangements during cardiac valve formation. Development 143, 2217-2227 (2016).
21. P. J. Scherz, J. Huisken, P. Sahai-Hernandez, D. Y. Stainier, High-speed imaging of developing heart valves reveals interplay of morphogenesis and function. Development 135, 1179-1187 (2008).
22. J. R. Hove, R. W. Koster, A. S. Forouhar, G. Acevedo-Bolton, S. E. Fraser, M. Gharib, Intracardiac fluid forces are an essential epigenetic factor for embryonic cardiogenesis. Nature 421, 172-177 (2003).
23. M. Wang, B. Y. Lin, S. Sun, C. Dai, F. Long, J. T. Butcher, Shear and hydrostatic stress regulate fetal heart valve remodeling through YAP-mediated mechanotransduction. Elife 12, (2023).
24. K. L. Pang, M. Parnall, S. Loughna, Effect of altered haemodynamics on the developing mitral valve in chick embryonic heart. J Mol Cell Cardiol 108, 114-126 (2017).
25. S. Kalogirou, N. Malissovas, E. Moro, F. Argenton, D. Y. Stainier, D. Beis, Intracardiac flow dynamics regulate atrioventricular valve morphogenesis. Cardiovasc Res 104, 49-60 (2014).
26. T. Bartman, E. C. Walsh, K. K. Wen, M. McKane, J. Ren, J. Alexander, P. A. Rubenstein, D. Y. Stainier, Early myocardial function affects endocardial cushion development in zebrafish. PLoS Biol 2, E129 (2004).
27. T. Haack, S. Abdelilah-Seyfried, The force within: endocardial development, mechanotransduction and signalling during cardiac morphogenesis. Development 143, 373-386 (2016).
28. L. M. Goddard, A. L. Duchemin, H. Ramalingan, B. Wu, M. Chen, S. Bamezai, J. Yang, L. Li, M. P. Morley, T. Wang, M. Scherrer-Crosbie, D. B. Frank, K. A. Engleka, S. C. Jameson, E. E. Morrisey, T. J. Carroll, B. Zhou, J. Vermot, M. L. Kahn, Hemodynamic Forces Sculpt Developing Heart Valves through a KLF2-WNT9B Paracrine Signaling Axis. Dev Cell 43, 274-289 e275 (2017).
29. F. Fontana, T. Haack, M. Reichenbach, P. Knaus, M. Puceat, S. Abdelilah-Seyfried, Antagonistic Activities of Vegfr3/Flt4 and Notchib Fine-tune Mechanosensitive Signaling during Zebrafish Cardiac Valvulogenesis. Cell Rep 32, 107883 (2020).
30. F. Wunnemann, A. Ta-Shma, C. Preuss, S. Leclerc, P. P. van Vliet, A. Oneglia, M. Thibeault, E. Nordquist, J. Lincoln, F. Scharfenberg, C. Becker-Pauly, P. Hofmann, K. Hoff, E. Audain, H. H. Kramer, W. Makalowski, A. Nir, S. S. Gerety, M. Hurles, J. Comes, A. Fournier, H. Osinska, J. Robins, M. Puceat, M. L. C. p. investigators, O. Elpeleg, M. P. Hitz, G. Andelfinger, Loss of ADAMT69 causes progressive non-syndromic heart valve disease. Not Genet 52, 40-47 (2020).
31. A. R. Chiplunkar, T. K. Lung, Y. Alhashem, B. A. Koppenhaver, F. N. Salloum, R. C. Kukreja, J. L. Haar, J. A. Lloyd, Kruppel-like factor 2 is required for normal mouse cardiac development. PLoS One 8, e54891 (2013).
32. S. J. Rasouli, M. El-Brolosy, A. T. Tsedeke, A. Bensimon-Brito, P. Ghanbari, H. M. Maischein, C. Kuenne, D. Y. Stainier, The flow responsive transcription factor Klf2 is required for myocardial wall integrity by modulating Fgf signaling. Elife 7, (2018).
33. D. Beis, T. Bartman, S. W. Jin, I. C. Scott, L. A. D'Amico, E. A. Ober, H. Verkade, J. Frantsve, H. A. Field, A. Wehman, H. Baier, A. Tallafuss, L. Bally-Cuif, J. N. Chen, D. Y. Stainier, B. Jungblut, Genetic and cellular analyses of zebrafish atrioventricular cushion and valve development. Development 132, 4193-4204 (2005).
34. K. A. Smith, A. K. Lagendijk, A. D. Courtney, H. Chen, S. Paterson, B. M. Hogan, C. Wicking, J. Bakkers, Transmembrane protein 2 (Tmem2) is required to regionally restrict atrioventricular canal boundary and endocardial cushion development. Development 138, 4193-4198 (2011).
35. D. MacGrogan, J. Munch, J. L. de la Pompa, Notch and interacting signalling pathways in cardiac development, disease, and regeneration. Not Rev Cardiol 15, 685-704 (2018).
36. G. Odelin, E. Faure, F. Kober, C. Maurel-Zaffran, A. Theron, F. Coulpier, B. Guillet, M. Bernard, J. F. Avierinos, P. Charnay, P. Topilko, S. Zaffran, Loss of Krox20 results in aortic valve regurgitation and impaired transcriptional activation of fibrillar collagen genes. Cardiovasc Res 104, 443-455 (2014).
37. G. Odelin, E. Faure, F. Coulpier, M. Di Bonito, F. Bajolle, M. Studer, J. F. Avierinos, P. Charnay, P. Topilko, S. Zaffran, Krox20 defines a subpopulation of cardiac neural crest cells contributing to arterial valves and bicuspid aortic valve. Development 145, (2018).
38. T. Banjo, J. Grajcarek, D. Yoshino, H. Osada, K. Y. Miyasaka, Y. S. Kida, Y. Ueki, K. Nagayama, K. Kawakami, T. Matsumoto, M. Sato, T. Ogura, Haemodynamically dependent valvulogenesis of zebrafish heart is mediated by flow-dependent expression of miR-21. Nat Commun 4, 1978 (2013).
39. M. Ghazvini-Boroujerdi, J. Clark, N. Narula, E. Palmatory, J. M. Connolly, S. DeFelice, J. Xu, B. Jian, S. Hazelwood, R. J. Levy, Transcription factor Egr-1 in calcific aortic valve disease. J Heart Valve Dis 13, 894-903 (2004).
40. A. Gaste, E. Bertrand, J.-F. Avierinos, V. Deplano, H. Ait-Oufella, S. Zaffran, Wall shear stress activation of EGR1 and KLF2 transcription in valvular cells is mediated by the ERK1/2 mitogen-activated protein kinase pathway. Archives of Cardiovascular Diseases Supplements 15, 220 (2023).
41. M. C. Hill, Z. A. Kadow, H. Long, Y. Morikawa, T. J. Martin, E. J. Birks, K. S. Campbell, J. Nerbonne, K. Lavine, L. Wadhwa, J. Wang, D. Turaga, I. Adachi, J. F. Martin, Integrated multi-omic characterization of congenital heart disease. Nature 608, 181-191 (2022).
42. J. Lotto, R. Cullum, S. Drissler, M. Arostegui, V. C. Garside, B. M. Fuglerud, M. Clement-Ranney, A. Thakur, T. M. Underhill, P. A. Hoodless, Cell diversity and plasticity during atrioventricular heart valve EMTs. Not Commun 14, 5567 (2023).
43. A. S. Forouhar, M. Liebling, A. Hickerson, A. Nasiraei-Moghaddam, H. J. Tsai, J. R. Hove, S. E. Fraser, M. E. Dickinson, M. Gharib, The embryonic vertebrate heart tube is a dynamic suction pump. Science 312, 751-753 (2006).
44. H. Anton, S. Harlepp, C. Ramspacher, D. Wu, F. Monduc, S. Bhat, M. Liebling, C. Paoletti, G. Charvin, J. B. Freund, J. Vermot, Pulse propagation by a capacitive mechanism drives embryonic blood flow. Development 140, 4426-4434 (2013).
45. A. Faucherre, H. Moha Ou Maati, N. Nasr, A. Pinard, A. Theron, G. Odelin, J. P. Desvignes, D. Salgado, G. Collod-Beroud, J. F. Avierinos, G. Lebon, S. Zaffran, C. Jopling, Piezo1 is required for outflow tract and aortic valve development. J Mol Cell Cardiol 143, 51-62 (2020).
46. F. Boselli, E. Steed, J. B. Freund, J. Vermot, Anisotropic shear stress patterns predict the orientation of convergent tissue movements in the embryonic heart. Development 144, 4322-4327 (2017).
47. H. Vignes, C. Vagena-Pantoula, M. Prakash, H. Fukui, C. Norden, N. Mochizuki, F. Jug, J. Vermot, Extracellular mechanical forces drive endocardial cell volume decrease during zebrafish cardiac valve morphogenesis. Dev Cell 57, 598-609 e595 (2022).
48. L. Amoasii, E. Sanchez-Ortiz, T. Fujikawa, J. K. Elmquist, R. Bassel-Duby, E. N. Olson, NURR1 activation in skeletal muscle controls systemic energy homeostasis. Proc Natl Acad Sci U S A 116, 11299-11308 (2019).
49. D. S. Peal, C. G. Burns, C. A. Macrae, D. Milan, Chondroitin sulfate expression is required for cardiac atrioventricular canal formation. Dev Dyn 238, 3103-3110 (2009).
50. D. Lai, X. Liu, A. Forrai, O. Wolstein, J. Michalicek, I. Ahmed, A. N. Garratt, C. Birchmeier, M. Zhou, L. Hartley, L. Robb, M. P. Feneley, D. Fatkin, R. P. Harvey, Neuregulin 1 sustains the gene regulatory network in both trabecular and nontrabecular myocardium. Circ Res 107, 715-727 (2010).
51. K. K. Marballi, K. Alganem, S. J. Brunwasser, A. Barkatullah, K. T. Meyers, J. M. Campbell, A. B. Ozols, R. E. McCullumsmith, A. L. Gallitano, Identification of activity-induced Egr3-dependent genes reveals genes associated with DNA damage response and schizophrenia. Transl Psychiatry 12, 320 (2022).
52. J. Lammi, J. Huppunen, P. Aarnisalo, Regulation of the osteopontin gene by the orphan nuclear receptor NURR1 in osteoblasts. Mol Endocrinol 18, 1546-1557 (2004).
53. D. Liu, I. Evans, G. Britton, I. Zachary, The zinc-finger transcription factor, early growth response 3, mediates VEGF-induced angiogenesis. Oncogene 27, 2989-2998 (2008).
54. J. Suehiro, T. Hamakubo, T. Kodama, W. C. Aird, T. Minami, Vascular endothelial growth factor activation of endothelial cells is mediated by early growth response-3. Blood 115, 2520-2532 (2010).
55. D. Zhao, S. Desai, H. Zeng, VEGF stimulates PKD-mediated CREB-dependent orphan nuclear receptor Nurr1 expression: role in VEGF-induced angiogenesis. Int J Cancer 128, 2602-2612 (2011).
56. L. Liaw, V. Lindner, S. M. Schwartz, A. F. Chambers, C. M. Giachelli, Osteopontin and beta 3 integrin are coordinately expressed in regenerating endothelium in vivo and stimulate Arg-Gly-Asp-dependent endothelial migration in vitro. Circ Res 77, 665-672 (1995).
57. D. R. Senger, S. R. Ledbetter, K. P. Claffey, A. Papadopoulos-Sergiou, C. A. Peruzzi, M. Detmar, Stimulation of endothelial cell migration by vascular permeability factor/vascular endothelial growth factor through cooperative mechanisms involving the alphavbeta3 integrin, osteopontin, and thrombin. Am J Pathol 149, 293-305 (1996).
58. D. Liu, H. Jia, D. I. Holmes, A. Stannard, I. Zachary, Vascular endothelial growth factor-regulated gene expression in endothelial cells: KDR-mediated induction of Egr3 and the related nuclear receptors Nur77, Nurr1, and Nor1. Arterioscler Thromb Vasc Biol 23, 2002-2007 (2003).
59. A. J. Sehnert, A. Huq, B. M. Weinstein, C. Walker, M. Fishman, D. Y. Stainier, Cardiac troponin T is essential in sarcomere assembly and cardiac contractility. Not Genet 31, 106-110 (2002).
60. H. Fukui, R. W. Chow, J. Xie, Y. Y. Foo, C. H. Yap, N. Mine, N. Mochizuki, J. Vermot, Bioelectric signaling and the control of cardiac cell identity in response to mechanical forces. Science 374, 351-354 (2021).
61. T. Juan, M. Bellec, B. Cardoso, H. Athea, N. Fukuda, M. Albu, S. Gunther, M. Looso, D. Y. R. Stainier, Control of cardiac contractions using Cre-lox and degron strategies in zebrafish. Proc Natl Acad Sci U S A 121, e2309842121 (2024).
62. E. Faure, E. Bertrand, A. Gaste, E. Plaindoux, V. Deplano, S. Zaffran, Side-dependent effect in the response of valve endothelial cells to bidirectional shear stress. Int J Cardiol 323, 220-228 (2021).
63. L. Miller, E. Birks, M. Guglin, H. Lamba, O. H. Frazier, Use of Ventricular Assist Devices and Heart Transplantation for Advanced Heart Failure. Circ Res 124, 1658-1678 (2019).
64. L. K. Truby, A. R. Garan, R. C. Givens, B. Wayda, K. Takeda, M. Yuzefpolskaya, P. C. Colombo, Y. Naka, H. Takayama, V. K. Topkara, Aortic Insufficiency During Contemporary Left Ventricular Assist Device Support: Analysis of the INTERMACS Registry. JACC Heart Fail 6, 951-960 (2018).
65. R. John, K. Mantz, P. Eckman, A. Rose, K. May-Newman, Aortic valve pathophysiology during left ventricular assist device support. J Heart Lung Transplant 29, 1321-1329 (2010).
66. A. G. Rose, S. J. Park, A. J. Bank, L. W. Miller, Partial aortic valve fusion induced by left ventricular assist device. Ann Thorac Surg 70, 1270-1274 (2000).
67. H. Hata, T. Fujita, H. Ishibashi-Ueda, T. Nakatani, J. Kobayashi, Pathological analysis of the aortic valve after long-term left ventricular assist device support. Eur J Cardiothorac Surg 46, 193-197 (2014).
68. J. Lotto, R. Cullum, S. Drissler, M. Arostegui, V. Garside, B. Fuglerud, A. Thakur, T. Underhill, P. Hoodless, Single-cell transcriptomics reveals diversity during heart valve epithelial-to-mesenchymal transitions. PREPRINT Research Square, (2022).
69. W. G. Tourtellotte, J. Milbrandt, Sensory ataxia and muscle spindle agenesis in mice lacking the transcription factor Egr3. Nat Genet 20, 87-91 (1998).
70. L. Gharibeh, H. Komati, Y. Bosse, M. Boodhwani, M. Heydarpour, M. Fortier, R. Hassanzadeh, J. Ngu, P. Mathieu, S. Body, M. Nemer, C. Bicuspid Aortic Valve, GATA6 Regulates Aortic Valve Remodeling, and Its Haploinsufficiency Leads to Right-Left Type Bicuspid Aortic Valve. Circulation 138, 1025-1038 (2018).
71. V. Garg, A. N. Muth, J. F. Ransom, M. K. Schluterman, R. Barnes, I. N. King, P. D. Grossfeld, D. Srivastava, Mutations in NOTCH 1 cause aortic valve disease. Nature 437, 270-274 (2005).
72. L. Luna-Zurita, B. G. Flores-Garza, D. Grivas, M. Siguero-Alvarez, J. L. de la Pompa, Cooperative Response to Endocardial Notch Reveals Interaction With Hippo Pathway. Circ Res 133, 1022-1039 (2023).
73. Y. S. Fan, V. M. Siu, Molecular cytogenetic characterization of a derivative chromosome 8 with an inverted duplication of 8p21.3-->p23.3 and a rearranged duplication of 8q24.13-->qter. Am J Med Genet 102, 266-271 (2001).
74. C. Gug, D. Stoicanescu, I. Mozos, L. Nussbaum, M. Cevei, D. Stambouli, A. G. Pavel, G. Doros, De novo 8p21.3--> p23.3 Duplication With t(4;8)(q35;p21.3) Translocation Associated With Mental Retardation, Autism Spectrum Disorder, and Congenital Heart Defects: Case Report With Literature Review. Front Pediatr 8, 375 (2020).
75. X. Li, Y. T. Ma, X. Xie, Y. N. Yang, X. Ma, Y. Y. Zheng, S. Pan, F. Liu, B. D. Chen, Association of Egr3 genetic polymorphisms and coronary artery disease in the Uygur and Han of China. Lipids Health Dis 13, 84 (2014).
76. R. Tsaryk, N. Yucel, E. V. Leonard, N. Diaz, O. Bondareva, M. Odenthal-Schnittler, Z. Arany, J. M. Vaquerizas, H. Schnittler, A. F. Siekmann, Shear stress switches the association of endothelial enhancers from ETV/ETS to KLF transcription factor binding sites. Sci Rep 12, 4795 (2022).
77. F. Fang, A. J. Shangguan, K. Kelly, J. Wei, K. Gruner, B. Ye, W. Wang, S. Bhattacharyya, M. E. Hinchcliff, W. G. Tourtellotte, J. Varga, Early growth response 3 (Egr-3) is induced by transforming growth factor-beta and regulates fibrogenic responses. Am J Pathol 183, 1197-1208 (2013).
78. V. Angeli, H. Y. Lim, Biomechanical control of lymphatic vessel physiology and functions. Cell Mol Immunol, (2023).
79. J. Chen, X. Liang, S. Zhang, S. Wang, S. P. Garcia, P. Yan, H. Yu, Z. Li, L. Liu, F. Zhang, W. Wei, H. Le, Y. Zhang, G. C. Yuan, S. Chen, Y. Chen, K. Sun, W. T. Pu, B. Zhang, Two faces of bivalent domain regulate VEGFA responsiveness and angiogenesis. Cell Death Dis 11, 75 (2020).
80. L. C. Dieterich, L. Ducoli, J. W. Shin, M. Detmar, Distinct transcriptional responses of lymphatic endothelial cells to VEGFR-3 and VEGFR-2 stimulation. Sci Data 4, 170106 (2017).
81. J. W. Shin, R. Huggenberger, M. Detmar, Transcriptional profiling of VEGF-A and VEGF-C target genes in lymphatic endothelium reveals endothelial-specific molecule-1 as a novel mediator of lymphangiogenesis. Blood 112, 2318-2326 (2008).
82. K. C. Rajan, N. R. Patel, A. Shenoy, J. P. Scallan, M. Y. Chiang, M. J. Galazo, S. M. Meadows, Zmiz1 is a novel regulator of lymphatic endothelial cell gene expression and function. bioRxiv, (2023).
83. K. J. Clark, D. Balciunas, H. M. Pogoda, Y. Ding, S. E. Westcot, V. M. Bedell, T. M. Greenwood, M. D. Urban, K. J. Skuster, A. M. Petzold, J. Ni, A. L. Nielsen, A. Patowary, V. Scaria, S. Sivasubbu, X. Xu, M. Hammerschmidt, S. C. Ekker, In vivo protein trapping produces a functional expression codex of the vertebrate proteome. Nat Methods 8, 506-515 (2011).
84. N. C. Chi, R. M. Shaw, S. De Val, G. Kang, L. Y. Jan, B. L. Black, D. Y. Stainier, Foxn4 directly regulates tbx2b expression and atrioventricular canal formation. Genes Dev 22, 734-739 (2008).
85. Y. Wang, M. S. Kaiser, J. D. Larson, A. Nasevicius, K. J. Clark, S. A. Wadman, S. E. Roberg-Perez, S. C. Ekker, P. B. Hackett, M. McGrail, J. J. Essner, Moesin1 and Ve-cadherin are required in endothelial cells during in vivo tubulogenesis. Development 137, 3119-3128 (2010).
86. L. Herwig, Y. Blum, A. Krudewig, E. Ellertsdottir, A. Lenard, H. G. Belting, M. Affolter, Distinct cellular mechanisms of blood vessel fusion in the zebrafish embryo. Curr Biol 21, 1942-1948 (2011).
87. A. Guerra, R. F. Germano, O. Stone, R. Arnaout, S. Guenther, S. Ahuja, V. Uribe, B. Vanhollebeke, D. Y. Stainier, S. Reischauer, Distinct myocardial lineages break atrial symmetry during cardiogenesis in zebrafish. Elife 7, (2018).
88. V. Uribe, R. Ramadass, D. Dogra, S. J. Rasouli, F. Gunawan, H. Nakajima, A. Chiba, S. Reischauer, N. Mochizuki, D. Y. R. Stainier, In vivo analysis of cardiomyocyte proliferation during trabeculation. Development 145, (2018).
89. Y. L. Ho, Y. H. Lin, I. J. Tsai, F. J. Hsieh, H. J. Tsai, In vivo assessment of cardiac morphology and function in heart-specific green fluorescent zebrafish. J Formos Med Assoc 106, 181-186 (2007).
90. K. Asakawa, M. L. Suster, K. Mizusawa, S. Nagayoshi, T. Kotani, A. Urasaki, Y. Kishimoto, M. Hibi, K. Kawakami, Genetic dissection of neural circuits by Tol2 transposon-mediated Gal4 gene and enhancer trapping in zebrafish. Proc Natl Acad Sci U S A 105, 1255-1260 (2008).
91. K. Hatta, H. Tsujii, T. Omura, Cell tracking using a photoconvertible fluorescent protein. Not Protoc 1, 960-967 (2006).
92. P. Han, J. Bloomekatz, J. Ren, R. Zhang, J. D. Grinstein, L. Zhao, C. G. Burns, C. E. Burns, R. M. Anderson, N. C. Chi, Coordinating cardiomyocyte interactions to direct ventricular chamber morphogenesis. Nature 534, 700-704 (2016).
93. J. Y. Bertrand, N. C. Chi, B. Santoso, S. Teng, D. Y. Stainier, D. Traver, Haematopoietic stem cells derive directly from aortic endothelium during development. Nature 464, 108-111 (2010).
94. L. E. Jao, S. R. Wente, W. Chen, Efficient multiplex biallelic zebrafish genome editing using a CRISPR nuclease system. Proc Natl Acad Sci U S A 110, 13904-13909 (2013).
95. A. Hruscha, P. Krawitz, A. Rechenberg, V. Heinrich, J. Hecht, C. Haass, B. Schmid, Efficient CRISPR/Cas9 genome editing with low off-target effects in zebrafish. Development 140, 4982-4987 (2013).
96. J. M. Welker, W. A. Wierson, M. P. Almeida, C. M. Mann, M. E. Torrie, Z. Ming, S. C. Ekker, K. J. Clark, D. L. Dobbs, J. J. Essner, M. McGrail, GeneWeld: Efficient Targeted Integration Directed by Short Homology in Zebrafish. Bio Protoc 11, e4100 (2021).
97. L. Burg, N. Palmer, K. Kikhi, E. S. Miroshnik, H. Rueckert, E. Gaddy, C. MacPherson Cunningham, K. Mattonet, S. L. Lai, R. Marin-Juez, R. B. Waring, D. Y. R. Stainier, D. Balciunas, Conditional mutagenesis by oligonucleotide-mediated integration of IoxP sites in zebrafish. PLoS Genet 14, e1007754 (2018).
98. W. A. Wierson, J. M. Welker, M. P. Almeida, C. M. Mann, D. A. Webster, M. E. Torrie, T. J. Weiss, S. Kambakam, M. K. Vollbrecht, M. Lan, K. C. McKeighan, J. Levey, Z. Ming, A. Wehmeier, C. S. Mikelson, J. A. Haltom, K. M. Kwan, C. B. Chien, D. Balciunas, S. C. Ekker, K. J. Clark, B. R. Webber, B. S. Moriarity, S. L. Solin, D. F. Carlson, D. L. Dobbs, M. McGrail, J. Essner, Efficient targeted integration directed by short homology in zebrafish and mammalian cells. Elife 9, (2020).
99. J. F. Watson, J. Garcia-Nafria, In vivo DNA assembly using common laboratory bacteria: A re-emerging tool to simplify molecular cloning. J Biol Chem 294, 15271-15281 (2019).
100. C. Thisse, B. Thisse, High-resolution in situ hybridization to whole-mount zebrafish embryos. Not Protoc 3, 59-69 (2008).
101. G. L. M. Boezio, S. Zhao, J. Gollin, R. Priya, S. Mansingh, S. Guenther, N. Fukuda, F. Gunawan, D. Y. R. Stainier, The developing epicardium regulates cardiac chamber morphogenesis by promoting cardiomyocyte growth. Dis Model Mech 16, (2023).
102. A. Theron, A. Touil, N. Resseguier, G. Collod-Beroud, G. Norscini, A. S. Simoni, G. Odelin, G. Habib, F. Collart, S. Zaffran, J. F. Avierinos, Clinical insights into a tertiary care center cohort of patients with bicuspid aortic valve. Int J Cardiovasc Imaging 38, 51-59 (2022).
103. H. Baumgartner, V. Falk, J. J. Bax, M. De Bonis, C. Hamm, P. J. Holm, B. lung, P. Lancellotti, E. Lansac, D. Rodriguez Munoz, R. Rosenhek, J. Sjogren, P. Tornos Mas, A. Vahanian, T. Walther, O. Wendler, S. Windecker, J. L. Zamorano, E. S. C. S. D. Group, 2017 ESC/EACTS Guidelines for the management of valvular heart disease. Eur Heart J 38, 2739-2791 (2017).
104. R. A. Nishimura, C. M. Otto, R. O. Bonow, B. A. Carabello, J. P. Erwin, 3rd, L. A. Fleisher, H. Jneid, M. J. Mack, C. J. McLeod, P. T. O'Gara, V. H. Rigolin, T. M. Sundt, 3rd, A. Thompson, 2017 AHA/ACC Focused Update of the 2014 AHA/ACC Guideline for the Management of Patients With Valvular Heart Disease: A Report of the American College of Cardiology/American Heart Association Task Force on Clinical Practice Guidelines. JAm Coll Cardiol 70, 252-289 (2017).
105. C. G. Burns, C. A. MacRae, Purification of hearts from zebrafish embryos. Biotechniques 40, 274, 276, 278 passim (2006).
106. K. Mattonet, F. W. Riemslagh, S. Guenther, K. D. Prummel, G. Kesavan, S. Hans, I. Ebersberger, M. Brand, A. Burger, S. Reischauer, C. Mosimann, D. Y. R. Stainier, Endothelial versus pronephron fate decision is modulated by the transcription factors Cloche/Npa9l, Tal1, and Lmo2. Sci Adv 8, eabn2082 (2022).
107. A. K. Cheung, J. M. Ko, H. L. Lung, K. W. Chan, E. J. Stanbridge, E. Zabarovsky, T. Tokino, L. Kashima, T. Suzuki, D. L. Kwong, D. Chua, S. W. Tsao, M. L. Lung, Cysteine-rich intestinal protein 2 (CRIP2) acts as a repressor of NF-kappaB-mediated proangiogenic cytokine transcription to suppress tumorigenesis and angiogenesis. Proc Natl Acad Sci U S A 108, 8390-8395 (2011).
108. X. Zhang, Z. Zhang, Q. Zhao, X. Lou, Rapid and Efficient Live Zebrafish Embryo Genotyping. Zebrafish 17, 56-58 (2020).
109. H. Schultheis, C. Kuenne, J. Preussner, R. Wiegandt, A. Fust, M. Bentsen, M. Looso, WlIsON: Web-based Interactive Omics VisualizatioN. Bioinformatics 35, 1055-1057 (2019).

## Claims

1. A method of producing cells having the phenotype of cardiac valve cells from precursor cells of valve cells comprising (a) introducing into said precursor cells early growth response 3 *(EGR3)* and/or a nucleic acid sequence encoding *EGR3.*

2. The method of claim 1, comprising prior to step (a)
(a') providing a cell population comprising precursor cells of valves cells, wherein said cell population has been obtained from a subject or has been derived from a cell line.

3. The method of claim 1 or 2, comprising after step (a)
(b) isolating cells having the phenotype of valve cells from the cell population obtained in step (a).

4. The method of anyone of claims 1 to 3, wherein said introducing into said precursor cells is effected by
(i) transforming said precursor cells with a nucleic acids molecule comprising the nucleic acid sequence encoding *EGR3;* and/or
(ii) microinjection, electroporation, lipofection and/or protein transduction of EGR3 into said precursor cells.

5. The method of claim 4, wherein the nucleic acid molecule is a vector.

6. The method of claim 5, wherein the vector is a lentiviral, adenoviral or adeno-associated vector.

7. The method of anyone of claims 1 to 6, wherein the precursor cells are embryonic stem cells, adult stem cells, iPS cells or endothelial cells (such as HUVEC cells).

8. The method of anyone of claims 1 to 7, wherein the cells having the phenotype of valve cells are **characterized by** the expression of
(i) with increasing preference **characterized by** the expression of one or more, two or more, three or more, four or more, and all five of SOX9, POSTN, HEY2, TGFB2 and HAND2; and/or
(ii) with increasing preference **characterized by** the expression of one or more, two or more, three or more, four or more, five or more, six or more, seven or more, eight or more, nine or more, ten or more, eleven or more, twelve or more and all thirteen of VIM, CD44, CD90, CD105, SPP1, α-SMA, SOX9, N-CAD, CDH11, VIM, COL1A1, COL3A1, and POSTN, and/or
(iii) with increasing preference **characterized by** the expression of one or more, two or more, three or more, four or more, five or more, six or more, seven or more, eight or more, nine or more, ten or more, eleven or more, twelve or more, thirteen or more, fourteen or more, fifteen or more, sixteen or more, and all seventenn of NFATc1, WBT2, BMP4, GATA5, NFKB1, CCND1, SMAD1, NPR3, CDH11, EDN1, MEF2C, SOX17, THSD1, EMCN, NOTCH4, DLL3/4 and JAG1.

9. The method of anyone of claims 1 to 7, wherein step (a) is carried out in the presence of one or more additional differentiation factors selected from the group consisting of BMP2, BMP10, bFGF, FGF2, FGF8, Wnt3a and VEGF.

10. A valve transplant comprising cells having the phenotype of valve cells obtained or obtainable by the method of anyone of claims 1 to 9.

11. Cells having the phenotype of valve cells obtained or obtainable by the method of anyone of claims 1 to 9, the valve transplant of claim 10 or a compound promoting the expression and/or the activity of early growth response 3 (EGR3) for use in treating a subject having a valve defect.

12. The compound for use of claim 11, wherein the compound is early growth response 3 (EGR3) and/or a nucleic acid sequence encoding *EGR3.*

13. The compound for use of claim 11, wherein the compound is
(a) an amino acid sequence which comprises or consists of the nucleic acid sequence of SEQ ID NO: 1 or 3, or an amino acid sequence which is at least 80%, preferably at least 90% and most preferably at least 95% identical thereto,
(b) a nucleic acid sequence which comprises or consists of the nucleic acid sequence of SEQ ID NO: 2 or 4, or a nucleic acid sequence which is at least 80%, preferably at least 90% and most preferably at least 95% identical thereto,
(c) an expression vector expressing the nucleic acid sequence as defined in (b), preferably under the control of an endocardial-specific and/or valve-cell promoter, and
(d) a host cell comprising the expression vector of (c),

14. The cells, valve transplant or compound for use of anyone of claim 11 to 13, wherein the subject having a valve defect has a congenital valve disease, preferably selected from bicuspid aortic valve, pulmonary atresia, mitral valve prolapse, pulmonary valve stenosis, or wherein the subject having a valve defect has an acquired valve disease selected from rheumatic heart disease, mitral valve regurgitation, aortic regurgitation, tricuspid regurgitation, and infective endocarditis.

15. A non-human animal model for a valve defect, wherein in the non-human animal the expression of *EGR3* is knocked-out or knocked-down.
